# EUROPEAN PATENT APPLICATION

(11) **EP 2 292 251 A1**
(43) Date of publication of application: **09.03.2011**
(21) Application number: 10010393.6
(22) Date of filing: 18.04.2002
(51) Int. Cl.: A61K 38/19, A61K 39/395, A61K 38/21, A61P 35/00

(54) **Combination therapy using anti-angiogenic agents and TNF-alpha**

(30) Priority: 24.04.2001 EP 01109981
(62) Divisional of application: 02745238.2
(71) Applicant: Merck Patent GmbH, 64293 Darmstadt (DE)
(72) Inventor: Grell, Matthias, Dr., 64291 Darmstadt (DE); Goodman, Simon, Dr., 64347 Griesheim (DE); Rueegg, Curzio, Prof., 1000 Lausanne 26 (CH)

(57) **Abstract**

The invention relates to a combination therapy for the treatment of tumors and tumor metastases comprising administration of anti-angiogenic agents and tumor necrosis factor alpha (TNFα) optionally together with other cytotoxic agents, such as interferon gamma (IFNγ) or chemotherapeutic agents such as cisplatin, or ErbB receptor inhibitors, such as anti-EGFR antibodies. The method and the pharmaceutical compositions comprising said agents can result in a synergistic potentiation of the tumor cell proliferation inhibition effect of each individual therapeutic agent, yielding more effective treatment than found by administering an individual component alone.

## Description

### TECHNICAL FIELD OF THE INVENTION:

The invention relates to a combination therapy for the treatment of tumors and tumor metastases comprising administration of anti-angiogenic agents and tumor necrosis factor alpha (TNFα) or a molecule having the biological activity of TNFα optionally together with other cytotoxic agents, such as interferon gamma (IFNγ) or chemotherapeutic agents such as cisplatin, or ErbB receptor inhibitors, such as anti-EGFR antibodies. The method and the pharmaceutical compositions comprising said agents can result in a synergistic potentiation of the tumor cell proliferation inhibition effect of each individual therapeutic agent, yielding more effective treatment than found by administering an individual component alone.

### BACKGROUND OF THE INVENTION:

Angiogenesis, also referred to as neovascularization, is a process of tissue vascularization that involves the growth of new developing blood vessels into a tissue. The process is mediated by the infiltration of endothelial cells and smooth muscle cells. The process is believed to proceed in any one of three ways: (1) The vessels can sprout from pre-existing vessels; (2) De novo development of vessels can arise from precursor cells (vasculogenesis); or (3) Existing small vessels can enlarge in diameter (Blood et al., 1990, Bioch. Biophys. Acta 1032, 89. Vascular endothelial cells are known to contain at least five RGD-dependent integrins, including the vitronectin receptor (αᵥβ₃ or αᵥβ₅), the collagen Types I and IV receptor, the laminin receptor, the fibronectin/laminin/collagen receptor and the fibronectin receptor (Davis et al., 1993, J. Cell. Biochem. 51, 206). The smooth muscle cell is known to contain at least six RGD-dependent integrins, including αᵥβ₃ and αᵥβ₅.
Angiogenesis is an important process in neonatal growth, but is also important in wound healing and in the pathogenesis of a large variety of clinically important diseases including tissue inflammation, arthritis, psoriasis, cancer, diabetic retinopathy, macular degeneration and other neovascular eye diseases. These clinical entities associated with angiogenesis are referred to as angiogenic diseases (Folkman et al., 1987, Science 235, 442).
Inhibition of cell adhesion in vitro using monoclonal antibodies immunospecific for various integrin α or β subunits have implicated the vitronectin receptor αᵥβ₃ in cell adhesion of a variety of cell types including microvascular endothelial cells (Davis et al., 1993, J. Cell. Biol. 51, 206).
Integrins are a class of cellular receptors known to bind extracellular matrix proteins, and therefore mediate cell-cell and cell-extracellular matrix interactions, referred generally to as cell adhesion events. The integrin receptors constitute a family of proteins with shared structural characteristics of noncovalent heterodimeric glycoprotein complexes formed of α and β subunits. The vitronectin receptor, named for its original characteristic of preferential binding to vitronectin, is now known to refer to three different integrins, designated αᵥβ₁, αᵥβ₃ and αᵥβ₅. αᵥβ₁ binds fibronectin and vitronectin. αᵥβ₃ binds a large variety of ligands, including fibrin, fibrinogen, laminin, thrombospondin, vitronectin and von Willebrand's factor. αᵥβ₅ binds vitronectin. It is clear that there are different integrins with different biological functions as well as different integrins and subunits having shared biological specificity. One important recognition site in a ligand for many integrins is the arginine-glycine-aspartic acid (RGD) tripeptide sequence. RGD is found in all of the ligands identified above for the vitronectin receptor integrins.
This RGD recognition site can be mimicked by linear and cyclic (poly)peptides that contain the RGD sequence. Such RGD peptides are known to be inhibitors or antagonists, respectively, of integrin function. It is important to note, however, that depending upon the sequence and structure of the RGD peptide, the specificity of the inhibition can be altered to target specific integrins. Various RGD polypeptides of varying integrin specificity have been described, for example, by Cheresh, et al., 1989, Cell 58, 945, Aumailley et al., 1991, FEBS Letts. 291, 50, and in numerous patent applications and patens (e.g. US patents 4,517,686, 4,578,079, 4,589,881, 4,614,517, 4,661,111, 4,792,525; EP 0770 622).

The generation of new blood vessels, or angiogenesis, plays a key role in the growth of malignant disease and has generated much interest in developing agents that inhibit angiogenesis (see, for example, Holmgren et al., 1995, Nature Medicine 1, 149; Folkman, 1995, Nature Medicine 1, 27; O'Reilly et. al., 1994, Cell 79, 315). The use of αᵥβ₃ integrin antagonists to inhibit angiogenesis is known in methods to inhibit solid tumor growth by reduction of the blood supply to the solid tumor (see, for example, US 5,753,230 and US 5,766,591, which describe the use of αᵥβ₃ antagonists such as synthetic polypeptides, monoclonal antibodies and mimetics of αᵥβ₃ that bind to the αᵥβ₃ receptor and inhibit angiogenesis). Methods and compositions for inhibiting αᵥβ₅ mediated angiogenesis of tissues using antagonists of the vitronectin receptor αᵥβ₅ are disclosed in WO 97/45447.
Angiogenesis is characterized by invasion, migration and proliferation of endothelial cells, processes that depend on cell interactions with extracellular matrix components. In this context, the integrin cell-matrix receptors mediate cell spreading and migration. The endothelial adhesion receptors of integrin αᵥβ₃ was shown to be a key player by providing a vasculature-specific target for anti-angiogenic treatment strategies (Brooks et al., 1994, Science 264, 569; Friedlander et. al., 1995, Science 270). The requirement for vascular integrin αᵥβ₃ in angiogenesis was demonstrated by several in vivo models where the generation of new blood vessels by transplanted human tumors was entirely inhibited either by systemic administration of peptide antagonists of integrin αᵥβ₃ and αᵥβ₅ , as indicated above, or, alternatively, by anti- αᵥβ₃ antibody LM609 (Brooks et al., 1994, Cell 79, 1157; ATCC HB 9537). This antibody blocks the αᵥβ₃ integrin receptor the activation of which by its natural ligands inhibits apoptosis of the proliferative angiogenic vascular cells and thereby disrupts the maturation of newly forming blood vessels, an event essential for the proliferation of tumors. Nevertheless, it was recently reported, that melanoma cells could form web-like patterns of blood vessels even in the absence of endothelial cells (Barinaga 1999, Science 285, 1475), implying that tumors might be able to circumvent some anti-angiogenic drugs which are only effective in the presence of endothelial tissue.

Numerous molecules stimulate endothelial proliferation, migration and assembly, including VEGF, Ang1 and bFGF, and are vital survival factors. VEGF (Vascular Endothelial Growth Factor) has been identified as a selective angiogenic growth factor that can stimulate endothelial cell mitogenesis. VEGF, in particular, is thought to be a major mediator of angiogenesis in a primary tumor and in ischemic ocular diseases. VEGF is a homodimer (MW : 46.000) that is an endothelial cell-specific angiogenic (Ferrara et al., 1992, Endocrin. Rev., 13, 18) and vasopermeable factor (Senger et al., 1986, Cancer Res., 465629) that binds to high-affinity membrane-bound receptors with tyrosine kinase activity (Jakeman et al., 1992, J. Clin. Invest., 89, 244). Human tumor biopsies exhibit enhanced expression of VEGF mRNAs by malignant cells and VEGF receptor mRNAs in adjacent endothelial cells. VEGF expression appears to be greatest in regions of tumors adjacent to vascular areas of necrosis. (for review see Thomas et al., 1996, J. Biol. Chem. 271 (2), 603; Folkman, 1995, Nature Medicine 1, 27). WO 97/45447 has implicated the αᵥβ₅ integrin in neovascularization, particularly, that induced by VEGF, EGF and TGF-α, and discloses that αᵥβ₅ antagonist can inhibit VEGF promoted angiogenesis. Effective anti-tumor therapies may also utilize targeting VEGF receptor for inhibition of angiogenesis using monoclonal antibodies. (Witte et al., 1998, Cancer Metastasis Rev. 17(2), 155). MAb DC-101 is known to inhibit angiogenesis of tumor cells.

Tyrosine kinases are a class of enzymes that catalyze the transfer of the terminal phosphate of adenosine triphosphate to tyrosine residues in protein substrates. Tyrosine kinases are believed, by way of substrate phosphorylation, to play critical roles in signal transduction for a number of cell functions. Though the exact mechanisms of signal transduction is still unclear, tyrosine kinases have been shown to be important contributing factors in cell proliferation, carcinogenesis and cell differentiation.

Tyrosine kinases can be categorized as receptor type or non-receptor type. Both receptor-type and non-receptor type tyrosine kinases are implicated in cellular signaling pathways leading to numerous pathogenic conditions, including cancer, psoriasis and hyperimmune responses. Many tyrosine kinases are involved in cell growth as well as in angiogenesis.

Receptor type tyrosine kinases have an extracellular, a transmembrane, and an intracellular portion, while non-receptor type tyrosine kinases are wholly intracellular. Receptor-linked tyrosine kinases are transmembrane proteins that contain an extracellular ligand binding domain, a transmembrane sequence, and a cytoplasmic tyrosine kinase domain. The receptor-type tyrosine kinases are comprised of a large number of transmembrane receptors with diverse biological activity. In fact, different subfamilies of receptor-type tyrosine kinases have been identified. Implicated tyrosine kinases include fibroblast growth factor (FGF) receptors, epidermal growth factor (EGF) receptors of the ErbB major class family, and platelet-derived growth factor (PDGF) receptors. Also implicated are nerve growth Factor (NGF) receptors, brain-derived neurotrophic Factor (BDNF) receptors, and neurotrophin-3 (NT-3) receptors, and neurotrophin-4 (NT-4) receptors.

One receptor type tyrosine kinase subfamily, designated as HER or ErbB subfamily, is comprised of EGFR (ErbB1), HER2 (ErbB2 or p185neu), HER3 (ErbB3), and HER4 (ErbB4 or tyro2). Ligands of this subfamily of receptors include epithelial growth factor (EGF), TGF-a, amphiregulin, HB-EGF, betacellulin and heregulin. The PDGF subfamily includes the FLK family which is comprised of the kinase insert domain receptor (KDR).

EGFR, encoded by the erbB1 gene, has been causally implicated in human malignancy. In particular, increased expression of EGFR has been observed in breast, bladder, lung, head, neck and stomach cancer as well as glioblastomas. Increased EGFR receptor expression is often associated with increased production of the EGFR ligand, transforming growth factor alpha (TGF-a), by the same tumor cells resulting in receptor activation by an autocrine stimulatory pathway (Baselga and Mendelsohn, Pharmac. Ther. 64:127-154 (1994)).
The EGF receptor is a transmembrane glycoprotein which has a molecular weight of 170.000, and is found on many epithelial cell types. It is activated by at least three ligands, EGF, TGF-α (transforming growth factor alpha) and amphiregulin. Both epidermal growth factor (EGF) and transforming growth factor-alpha (TGF-a) have been demonstrated to bind to EGF receptor and to lead to cellular proliferation and tumor growth. These growth factors do not bind to HER2 (Ulrich and Schlesinger, 1990, Cell 61, 203). In contrary to several families of growth factors, which induce receptor dimerization by virtue of their dimeric nature (e.g. PDGF) monomeric growth factors, such as EGF, contain two binding sites for their receptors and, therefore, can cross-link two neighboring EGF receptors (Lemmon et al., 1997, EMBO J. 16, 281). Receptor dimerization is essential for stimulating of the intrinsic catalytic activity and for the auto-phosphorylation of growth factor receptors. It should be remarked that receptor protein tyrosine kinases (PTKs) are able to undergo both homo- and heterodimerization.
It has been demonstrated that anti-EGF receptor antibodies while blocking EGF and TGF-a binding to the receptor can inhibit tumor cell proliferation. In view of these findings, a number of murine and rat monoclonal antibodies against EGF receptor have been developed and tested for their ability inhibit the growth of tumor cells in vitro and in vivo (Modjtahedi and Dean, 1994, J. Oncology 4, 277*).* Humanized monoclonal antibody 425 (hMAb 425, US 5,558,864; EP 0531 472) and chimeric monoclonal antibody 225 (cMAb 225, US 4,943,533 and EP 0359 282), both directed to the EGF receptor, have shown their efficacy in clinical trials. The C225 antibody was demonstrated to inhibit EGF-mediated tumor cell growth in vitro and inhibit human tumor formation in vivo in nude mice. The antibody, moreover, appeared to act, above all, in synergy with certain chemotherapeutic agents (i.e., doxorubicin, adriamycin, taxol, and cisplatin) to eradicate human tumors in vivo in xenograft mouse models. Ye et al. (1999, Oncogene 18, 731) have reported that human ovarian cancer cells can be treated successfully with a combination of both cMAb 225 and humanized MAb 4D5 which is directed to the HER2 receptor.

The second member of the ErbB family, HER2 (ErbB2 or p185*neu*), was originally identified as the product of the transforming gene from neuroblastomas of chemically treated rats. The activated form of the neu proto-oncogene results from a point mutation (valine to glutamic acid) in the transmembrane region of the encoded protein. Amplification of the human homolog of *neu* is observed in breast and ovarian cancers and correlates with a poor prognosis (Slamon et al., Science, 235: 177-182 (1987); Slamon et al., Science, 244:707-7 12 (1989); US 4,968,603). ErbB2 (HER2) has a molecular weight of about 185.000, with considerable homology to the EGF receptor (HER1), although a specific ligand for HER2 has not yet been clearly identified so far.
The antibody 4D5 directed to the HER2 receptor, was further found to sensitize ErbB2-overexpressing breast tumor cell lines to the cytotoxic effects of TNFα (US 5,677,171 ). A recombinant humanized version of the murine anti-ErbB2 antibody 4D5 (huMAb4D5-8, rhuMAb HER2 or HERCEPTIN® ; US 5,821,337) is clinically active in patients with ErbB2-overexpressing metastatic breast cancers that have received extensive prior anti-cancer therapy (Baselga et al., J. Clin. Oncol. 14:737-744 (1996)). HERCEPTIN ® received marketing approval in 1998 for the treatment of patients with metastatic breast cancer whose tumors overexpress the ErbB2 protein.

TNFα belongs to a large family of molecules including important cytokines such as Fas ligand, CD40 ligand, TRAIL, lymphotoxin and others (Locksley et al., 2001, Cell 104:487-501). Besides being released from many cell types, TNFα also exists in a cell-membrane bound, higher molecular weight form on cells, and this form also appears to mediate a variety of biological effects. TNFα is thought to have few roles in normal development and physiology; however, it exerts harmful and destructive effects on many tissues in many disease states (Tracey et al., Ann. Rev. Med 1994; 45:491). Disease states in which TNFα has been shown to exert a major role include septic shock syndrome, cancer cachexia, rheumatoid arthritis, etc.
Human TNFα was first purified in 1985 (see Aggarwal et al., J Biol. Chem. 1985, 260, 2345-2354). Soon after, the molecular cloning of the TNF cDNA and the cloning of the human TNF locus were accomplished (Pennica et al., Nature 1984, 312, 124-729; Wang et al., Nature 1985, 313, 803-806). TNFα is a trimeric 17 KDa polypeptide mainly produced by macrophages. This peptide is initially expressed as a 26 KDa transmembrane protein from which the 17 KDa subunit is cleaved and released proteolytic cleavage. TNFα is typically produced by various cells: for example, activated macrophages and fibroblasts. TNFα has been reported to induce a lot of diverse factors. TNFα has also been also reported to participate, either directly or indirectly, in various diseases such as infectious diseases, auto-immune diseases such as systemic lupus erythematosus (SLE) and arthritis, AIDS, septicemia, and certain types of infections.
TNFα and inflammatory response infection and tissue injury induce a cascade of biochemical changes that trigger the onset of perplexing reactions of the immune system, collectively referred to as inflammatory response. The evolution of this response is based, at least in part, on local vasodilation or enhancing vascular permeability and activation of the vascular endothelium, which allows white blood cells to efficiently circulate and migrate to the damaged site, thereby increasing their chances to bind to and destroy any antigens. The vascular endothelium is thought to then be activated or inflamed. Generally, inflammation is a welcomed immune response to a variety of unexpected stimuli, and as such it exhibits rapid onset and short duration (acute inflammation). Its persistent or uncontrolled activity (chronic inflammation) has, however, detrimental effects to the body and results in the pathogenesis of several immune diseases, such as: septic shock, rheumatoid arthritis, inflammatory bowel diseases and congestive heart failure (see "TNF and TNF receptor superfamily" in "Cytokines and cytokine receptors", Bona and Revillard (Eds.), Harvard Academic Publishers, Amsterdam 2000, pages 118-148).

TNFα as well as many other cytokines are secreted by macrophages shortly after the initiation of the inflammatory response and induce coagulation, increase the vascular permeability and activate the expression of adhesion molecules on vascular endothelial cells.
TNFα is neither completely beneficial nor completely destructive to the host. Thus, TNFα is a potent modulator of endothelial cell function. Depending on the vascular context it promotes inflammation by inducing endothelial cell activation and survival or it causes tissue necrosis by inducing endothelial cell apoptosis and vascular disruption (Pober, J. S., Pathol Biol (Paris) 46, 159-163. (1998); Aggarwal, & Natarajan, Eur. Cytokine Netw. 7, 93-124 (1996)). Many intracellular signaling pathways mediating these two divergent responses have been characterized (Wallach et al., Annual Review of Immunology 17, 331-367 (1999)), but the extracellular cues that determine whether endothelial cells exposed to TNFα will survive or die, have remained elusive.
Rather, balance of its production and regulation is maintained to ensure that the host can effectively react to invading microorganisms without compromising host well-being in the process. Being a mediator of inflammation, TNFα helps the body in its fight against bacterial infections and tissue injuries by boosting an appropriate immune response. However, its overproduction leads to chronic inflammation, has detrimental effects to the body and plays a major role in the pathogenesis of several diseases.
IFNγ is a potent enhancer of TNFα (Dealtry et al., Eur J Immunol 17, 689-693, (1987)). In case where TNFα causes cell apoptosis, activation of NF-κB, a transcription factor that promotes cell survival, may suppress TNFα -induced apoptosis (Van Antwerp et al., Science 274, 787-789 (1996)).
TNFα induces a broad variety of cellular signals leading to cellular responses such as proliferation, activation, differentiation but also to programmed cell death. Cellular signaling to TNFα can be categorized into early responses like activation of kinases, phosphatases, lipases, proteases and transcription factors, and late responses, and thus more indirect responses like pertubation of the electron transport chain in the mitochondria, radical production, oxide production and the release of various substances. Many of the early cellular responses, such as the recruitment of death domain containing adaptor proteins, activation of NFκB or caspase activation, are also initiated by binding of other members of the TNF ligand family to their respective receptors. Accordingly, molecules like lymphotoxin, Fas ligand or TRAIL can act redundantly with TNF (Grell and Clauss, I.c.).
Integrin-mediated adhesion to the extracellular matrix (ECM) is essential for the survival of most cells, including endothelial cells. For example vascular integrin αVβ3 promotes proliferation and survival of angiogenic endothelial cells and αVβ3 antagonists induce apoptosis of angiogenic endothelial cell and suppress angiogenesis (Brooks et al., Cell 79, 1157-1164 (1994). Several of the biochemical events associated with integrin-mediated cell survival, including activation of PI 3-K/AKT (Khwaja et al., Embo Journal 16, 2783-2793 (1997)) and NF-_{K}B (Scatena et al., J Cell Biol 141, 1083-1093 (1998)) signaling pathways, have been identified. Besides integrins, the cell-cell adhesion molecules PECAM-1 and VE Cadherin also promote endothelial cell survival (Bird et al. J Cell Sci 112, 1989-1997 (1999); Carmeliet et al. Cell 98, 147-157 (1999)).
TNF is cytotoxic for some tumor cell lines, but most of them are hardly affected in growth. It is therefore unlikely that the antitumoral effects of TNF in some animal models (Balkwill et al., Cancer Res. 46: 3990-3993 (1986)) are due to direct action of the cytokine on tumor cells. In several studies it has been shown that host mediated mechanisms are involved in TNF triggered tumor regression (Manda et al., Cancer Res. 47: 3707-3711 (1987)). Accumulating data indicate that hemorrhagic necrosis of tumors by TNF is initiated at the endothelial cell level of the intratumoral vessels (Havell et al., J. Exp. Med. 167: 1967-1985 (1988)).
The results of clinical TNF studies in cancer patients are, by and large, disappointing (reviewed by Haranaka, J. Biol. Response Mod. 7: 525-534 (1988)). Generally, the antitumoral effects of TNF are limited by considerable side effects. One approach to limit the side effects of TNF has been the generation of TNF mutants displaying either TNF receptor type 1-specific activities or different pharmacodynamic properties (Brouckaert et al., Circ. Shock 43: 185-190 (1994); Eggermont, Anticancer Res. 18: 3899-3905 (1998); Lucas et al., Int. J. Cancer 15: 543-549 (2001)). Recently progress has been achieved in patients suffering from melanomas or sarcomas of the extremities. Significant beneficial effects could be obtained by isolated perfusion technique. Extreme dosages of TNF up to 4 mg are used in combination with cytostatics or IFN (Lienard et al., J. Clin. Oncol. 10: 52-60 (1992)). Local responses include acute softening and redness of the tumor associated with a strong inflammatory response, similar to TNF mediated anti-tumoral effects in murine systems.

It was shown that this treatment to patients with metastatic melanoma of the limbs selectively disrupts the tumor vasculature but leaves quiescent vessels intact. This effect is associated with TNF and IFNγ-induced suppression of integrin αVβ3-function in endothelial cells *in vitro* and induction of endothelial cell apoptosis *in vivo* (Ruegg et al, Nature Med 4, 408-414 (1998)). These results demonstrate that TNF in combination with additional therapeutic agents can be clinically very effective in the treatment of some tumors, provided systemic toxicity can be controlled.

The present invention describes now that molecules contributed to angiogenesis such as integrins, may have, while modulating TNFα activity, direct implications to the clinical use of TNFα as anti-cancer agent. Coadministration of anti-angiogenic agents together with TNFα, preferably integrin antagonists, may selectively sensitize angiogenesis receptor bearing endothelial cells to the apoptotic activity of TNF resulting in an improved disruption of tumor vessels. Therefore, this combination therapy can facilitate the reduction of TNF doses avoiding the systemic side effects of TNF.

### SUMMARY OF THE INVENTION

The present inventions describes for the first time the new concept in tumor therapy to administer to an individual an agent that blocks or inhibits angiogenesis together with TNFα, TNF mutants or TNF-like molecules. Optionally the composition according to this invention comprises further therapeutically active compounds, preferably selected from the group consisting of cytotoxic agents, chemotherapeutic agents and inhibitors or antagonists of the ErbB receptor tyrosine kinase family, such as described below in more detail.
Thus, the invention relates to pharmaceutical compositions comprising as preferred anti-angiogenic agents, integrin (receptor) antagonists and TNFα, TNF mutants or TNF-like molecules in a therapeutically effective amount. More specifically, the invention relates to pharmaceutical compositions comprising linear or cyclic RGD peptides and TNFα optionally together with IFNγ. The preferred composition according to the invention comprises the cyclic peptide cyclo-(Arg-Gly-Asp-DPhe-NMe-Val), TNFα and IFNγ. According to this invention said therapeutically active agents may also be provided by means of a pharmaceutical kit comprising a package comprising one or more anti-angiogenic agents, TNFα, and, optionally, one or more cytotoxic /chemotherapeutic agents / anti-ErbB agents in single packages or in separate containers.

The invention relates, more specifically, to a combination therapy comprising the application and administration, respectively, of two or more molecules, wherein at least one molecule has an angiogenesis inhibitory activity and the other one is TNFα. However, the invention relates, furthermore, to a combination therapy comprising the administration of only one (fusion) molecule, having anti-angiogenic activity and TNFα activity, optionally together with one or more cytotoxic / chemotherapeutic agents. For example, a fusion protein consisting essentially of cyclo-(Arg-Gly-Asp-DPhe-NMe-Val) fused directly or via a linker molecule to TNFα may be applied to a patient. Another example is an anti-integrin antibody, such as LM609 as described below, which is fused at the C-terminal of its Fc portion to TNFα. A further example is a bispecific antibody fused to TNFα, wherein on specificity is directed to an integrin receptor or a VEGF receptor and the other one is directed to the EGF receptor.
Principally, the administration can be accompanied by radiation therapy, wherein radiation treatment can be done substantially concurrently or before or after the drug administration. The administration of the different agents of the combination therapy according to the invention can also be achieved substantially concurrently or sequentially. Tumors, bearing receptors on their cell surfaces involved in the development of the blood vessels of the tumor, may be successfully treated by the combination therapy of this invention.

It is known that tumors elicit alternative routes for their development and growth. If one route is blocked they often have the capability to switch to another route by expressing and using other receptors and signaling pathways. Therefore, the pharmaceutical combinations of the present invention may block several of such possible development strategies of the tumor and provide consequently various benefits. The combinations according to the present invention are useful in treating and preventing tumors, tumor-like and neoplasia disorders and tumor metastases which are described below in more detail. Preferably, the different combined agents of the present invention are administered in combination at a low dose, that is, at a dose lower than has been conventionally used in clinical situations. A benefit of lowering the dose of the compounds, compositions, agents and therapies of the present invention administered to an individaul includes a decrease in the incidence of adverse effects associated with higher dosages. For example, by the lowering the dosage of a chemotherapeutic agent such as methotrexate, a reduction in the frequency and the severity of nausea and vomiting will result when compared to that observed at higher dosages. By lowering the incidence of adverse effects, an improvement in the quality of life of a cancer patient is contemplated. Further benefits of lowering the incidence of adverse effects include an improvement in patient compliance, a reduction in the number of hospitalizations needed for the treatment of adverse effects, and a reduction in the administration of analgesic agents needed to treat pain associated with the adverse effects. Alternatively, the methods and combination of the present invention can also maximize the therapeutic effect at higher doses.
The combinations according to the inventions show an astonishing synergetic effect. In administering the combination of drugs real tumor shrinking and disintegration could be observed during clinical studies while no significant adverse drug reactions were detectable.

In detail the invention refers to:
- a pharmaceutical composition comprising in an therapeutically effective amount at least (i) one anti-angiogenic agent and (ii) tumor necrosis factor alpha (TNFα) or a molecule having the biological activity of TNFα, optionally together with a pharmaceutically acceptable carrier, excipient or diluent;
- a corresponding pharmaceutical composition, wherein said anti-angiogenic agent is an integrin (receptor) inhibitor/antagonist or a VEGF (receptor) inhibitor/antagonist;
- a corresponding pharmaceutical composition, wherein said integrin receptor inhibitor/antagonist is an RGD- containing linear or cyclic peptide;
- a corresponding pharmaceutical composition, wherein said RGD-containing peptide is cyc/o-(Arg-Gly-Asp-DPhe-NMeVal);
- a corresponding pharmaceutical composition, wherein said anti-angiogenic agent is an antibody or an immunotherapeutically active fragment thereof, binding to an integrin receptor or VEGF receptor;
- a corresponding pharmaceutical composition, wherein said anti-angiogenic agent and TNFα are linked together to form one fusion molecule;
- a corresponding pharmaceutical composition, further comprising at least one cytotoxic and or chemotherapeutic agent;
- a corresponding pharmaceutical composition, wherein said cytotoxic agent is interferon gamma (IFNγ) and / or another effective cytokine;
- a corresponding pharmaceutical composition, wherein said chemotherapeutic compound is selected from the group consisting of: cisplatin, doxorubicin, gemcitabine, docetaxel, paclitaxel (taxol), bleomycin;
- a corresponding pharmaceutical composition, further comprising an inhibitor or antagonist of the ErbB receptor tyrosine kinase family;
- a corresponding pharmaceutical composition, wherein said inhibitor is an anti- EGFR antibody, an anti-HER2 antibody or an immunotherapeutically active fragment thereof;
- a pharmaceutical kit comprising a package comprising (i) at least one anti-angiogenic agent, preferably an integrin receptor inhibitor / antagonist, (ii) TNFα and optionally (iii) a further cytotoxic and/or chemotherpeutic agent;
- a correspondingly preferred pharmaceutical kit comprising
   (i) cyclo(Arg-Gly-Asp-DPhe-NMeVal), (ii) TNFα and (iii) IFNγ and optionally (iii) a further cytotoxic and/or chemotherpeutic agent and / or an inhibitor or antagonist of the ErbB receptor tyrosine kinase family;
- a corresponding pharmaceutical kit, wherein said pharmaceutically active agents are provided in separate containers in said package;
- the use of said pharmaceutical composition as defined above and in the claims, for the manufacture of a medicament or a composition of medicaments to treat tumors and tumor metastases; and
- a method for treating tumors or tumor metastases in an individual comprising administering to said individual simultaneously or sequentially a therapeutically effective pharmaceutical compositions as defined above;

### DETAILED DESCRIPTION OF THE INVENTION

If not otherwise pointed out the terms and phrases used in this invention have the meanings and definitions as given below. Moreover, these definitions and meanings describe the invention in more detail, preferred embodiments included.

**"Biological molecules"** include natural or synthetic molecules having, as a rule, a molecular weight greater than approximately 300, and are preferably poly- and oligosaccharides, oligo- and polypeptides, proteins, peptides, poly- and oligonucleotides as well as their glycosylated lipid derivatives. Most typically, biological molecules include immunotherapeutic agents, above all antibodies or fragments thereof, or functional derivatives of these antibodies or fragments including fusion proteins.

A **"receptor"** or **"receptor molecule"** is a soluble or membrane bound /associated protein or glycoprotein comprising one or more domains to which a ligand binds to form a receptor-ligand complex. By binding the ligand, which may be an agonist or an antagonist the receptor is activated or inactivated and may initiate or block pathway signaling.

By **" ligand"** or **"receptor ligand"** is meant a natural or synthetic compound which binds a receptor molecule to form a receptor-ligand complex. The term ligand includes agonists, antagonists, and compounds with partial agonist/antagonist action. According to the specific field of this invention the term includes, above all, TNF-like ligands.

The term **"TNF**α**"** as used herein, includes, if not specifically restricted, all kinds of TNF molecules and molecules having the biological activity of TNFα, including natural and synthetic, peptidic or non-peptidic TNF mutants, variants or'TNF-like ligands. Preferably, the term means natural peptidicTNFα.

An **"agonist"** or **"receptor agonist"** is a natural or synthetic compound which binds the receptor to form a receptor-agonist complex by activating said receptor and receptor-agonist complex, respectively, initiating a pathway signaling and further biological processes.

By **"antagonist"** or **"receptor antagonist"** is meant a natural or synthetic compound that has a biological effect opposite to that of an agonist. An antagonist binds the receptor and blocks the action of a receptor agonist by competing with the agonist for receptor. An antagonist is defined by its ability to block the actions of an agonist. A receptor antagonist may be also an antibody or an immunotherapeutically effective fragment thereof. Preferred antagonists according to the present invention are cited and discussed below.

The term **"therapeutically effective"** or **"therapeutically effective amount"** refers to an amount of a drug effective to treat a disease or disorder in a mammal. In the case of cancer, the therapeutically effective amount of the drug may reduce the number of cancer cells; reduce the tumor size; inhibit (i.e., slow to some extent and preferably stop) cancer cell infiltration into peripheral organs; inhibit (i.e., slow to some extent and preferably stop) tumor metastasis; inhibit, to some extent, tumor growth; and/or relieve to some extent one or more of the symptoms associated with the cancer. To the extent the drug may prevent growth and/or kill existing cancer cells, it may be cytostatic and/or cytotoxic. For cancer therapy, efficacy can, for example, be measured by assessing the time to disease progression (TTP) and/or determining the response rate (RR).

The term **"immunotherapeutically effective"** refers to biological molecules which cause an immune response in a mammal. More specifically, the term refers to molecules which may recognize and bind an antigen. Typically, antibodies, antibody fragments and antibody fusion proteins comprising their antigen binding sites (complementary determining regions, CDRs) are immunotherapeutically effective.

The term **"prodrug"** as used in this application refers to a precursor or derivative form of a pharmaceutically active substance that is less cytotoxic to tumor cells compared to the parent drug and is capable of being enzymatically activated or converted into the more active parent form (see, e.g. "Prodrugs in Cancer Chemotherapy", Biochemical Society Transactions, 14, pp. 375-382,615th Meeting Belfast (1986)).

An **"anti-angiogenic agent"** refers to a natural or synthetic compound which blocks, or interferes with to some degree, the development of blood vessels. The anti-angiogenic molecule may, for instance, be a biological molecule that binds to and blocks an angiogenic growth factor or growth factor receptor. The preferred anti-angiogenic molecule herein binds to an receptor, preferably to an integrin receptor or to VEGF receptor. The term includes according to the invention also a prodrug of said angiogenic agent.
There are a lot of molecules having different structure and origin which elicit anti-agiogenic properties. Most relevant classes of angiogenesis inhibitong or blocking agents which are suitable in this invention, are, for example:
(i) anti-mitotics such as flurouracil, mytomycin-C, taxol;
(ii) estrogen metabolites such as 2-methoxyestradiol;
(iii) matrix metalloproteinase (MMP) inhibitors, which inhibit zinc metalloproteinases (metalloproteases) (e.g. betimastat, BB16, TIMPs, minocycline, GM6001, or those described in "Inhibition of Matrix Metalloproteinases: Therapeutic Applications" (Golub, Annals of the New York Academy of Science, Vol. 878a; Greenwald, Zucker (Eds.), 1999);
(iv) anti-angiogenic multi-functional agents and factors such as IFNα (US 4,530,901; US 4,503,035; 5,231,176); angiostatin and plasminogen fragments (e.g. kringle 1-4, kringle 5, kringle 1-3 (O'Reilly, M. S. et al., Cell (Cambridge, Mass.) 79(2): 315-328, 1994*;* Cao et al., J. Biol. Chem. 271: 29461-29467, 1996; Cao et al., J. Biol Chem 272: 22924-22928, 1997); endostatin (O'Reilly, M. S. et al., Cell 88(2), 277, 1997 and WO 97/15666), thrombospondin (TSP-1; Frazier,1991, Curr Opin Cell Biol 3(5): 792); platelet factor 4 (PF4);
(v) plasminogen activator/urokinase inhibitors;
(vi) urokinase receptor antagonists;
(vii) heparinases;
(viii) fumagillin analogs such as TNP-470;
(ix) tyrosine kinase inhibitors such as SUI 01 (many of the above and below - mentioned ErbB receptor antagonists (EGFR / HER2 antagonists) are also tyrosine kinase inhibitors, and may show, therefore anti-EGF receptor blocking activity which results in inhibiting tumor growth, as well as anti-angiogenic activity which results in inhibiting the development of blood vessels and endothelial cells, respectively);
(x) suramin and suramin analogs;
(xi) angiostatic steroids;
(xii) VEGF and bFGF antagonists;
(xiii) VEGF receptor antagonists such as anti-VEGF receptor antibodies (DC-101);
(xiv) flk-1 and flt-1 antagonists;
(xv) cyclooxxyqenase-II inhibitors such as COX-II;
(xvi) integrin antagonists and integrin receptor antagonists such as αv antagonists and αv receptor antagonists, for example, anti-αv receptor antibodies and RGD peptides. Integrin (receptor) antagonists are preferred according to this invention.

The term **"integrin antagonists** / **inhibitors" or "integrin receptor antagonists** / **inhibitors"** refers to a natural or synthetic molecule that blocks and inhibit an integrin receptor. In some cases, the term includes antagonists directed to the ligands of said integrin receptors (such as for αᵥβ₃: vitronectin, fibrin, fibrinogen, von Willebrand's factor, thrombospondin, laminin; for αᵥβ₅: vitronectin; for αᵥβ₁: fibronectin and vitronectin; for αᵥβ₆: fibronectin). Antagonists directed to the integrin receptors are preferred according to the invention. Integrin (receptor) antagonists may be natural or synthetic peptides, non-peptides, peptidomimetica, immunoglobulins, such as antibodies or functional fragments thereof, or immunoconjugates (fusion proteins). Preferred integrin inhibitors of the invention are directed to receptor of αᵥ integrins (e.g. αᵥβ₃, αᵥβ₅, αᵥβ₆ and sub-classes). Preferred integrin inhibitors are αᵥ antagonists, and in particular αᵥβ₃ antagonists. Preferred αᵥ antagonists according to the invention are RGD peptides, peptidomimetic (non-peptide) antagonists and anti-integrin receptor antibodies such as antibodies blocking αᵥ receptors.
Exemplary, non-immunological αᵥβ₃ antagonists are described in the teachings of US 5,753,230 and US 5,766,591. Preferred antagonists are linear and cyclic RGD-containing peptides. Cyclic peptides are, as a rule, more stable and elicit an enhanced serum half-life. The most preferred integrin antagonist of the invention is, however, cyclo-(Arg-Gly-Asp-DPhe-NMeVal) (EMD 121974, Cilengitide^{®}, Merck KgaA, Germany; EP 0770 622) which is efficacious in blocking the integrin receptors αᵥβ₃, αᵥβ₁, αᵥβ₆, αᵥβ₈, α_{IIb}β₃.
Suitable peptidyl as well as peptidomimetic (non-peptide) antagonists of the αᵥβ₃ / αᵥβ₅ / αᵥβ₆ integrin receptor have been described both in the scientific and patent literature. For example, reference is made to Hoekstra and Poulter, 1998, Curr. Med. Chem. 5, 195; WO 95/32710; WO 95/37655; WO 97/01540; WO 97/37655; WO 97/45137; WO 97/41844; WO 98/08840; WO 98/18460; WO 98/18461; WO 98/25892; WO 98/31359; WO 98/30542; WO 99/15506; WO 99/15507; WO 99/31061; WO 00/06169; EP 0853 084; EP 0854 140; EP 0854 145; US 5,780,426; and US 6,048,861. Patents that disclose benzazepine, as well as related benzodiazepine and benzocycloheptene αᵥβ₃ integrin receptor antagonists, which are also suitable for the use in this invention, include WO 96/00574, WO 96/00730, WO 96/06087, WO 96/26190, WO 97/24119, WO 97/24122, WO 97/24124, WO 98/15278, WO 99/05107, WO 99/06049, WO 99/15170, WO 99/15178, WO 97/34865, WO 97/01540, WO 98/30542, WO 99/11626, and WO 99/15508. Other integrin receptor antagonists featuring backbone conformational ring constraints have been described in WO 98/08840; WO 99/30709; WO 99/30713; WO 99/31099; WO 00/09503; US 5,919,792; US 5,925,655; US 5,981,546; and US 6,017,926. In US 6,048,861 and WO 00/72801 a series of nonanoic acid derivatives which are potent αᵥβ₃ integrin receptor antagonists were disclosed. Other chemical small molecule integrin antagonists (mostly vitronectin antagonists) are described in WO 00/38665. Other αᵥβ₃ receptor antagonists have been shown to be effective in inhibiting angiogenesis. For example, synthetic receptor antagonists such as (S)-10,11-Dihydro-3-[3-(pyridin-2-ylamino)-1-propyloxy]-5H-dibenzo[a,d]cycloheptene-10-acetic acid (known as SB-265123) have been tested in a variety of mammalian model systems. (Keenan et al., 1998, Bioorg. Med. Chem. Lett. 8(22), 3171; Ward et al., 1999, Drug Metab. Dispos. 27(11),1232). Assays for the identification of integrin antagonists suitable for use as an antagonist are described, e.g. by Smith et al., 1990, J. Biol. Chem. 265, 12267, and in the referenced patent literature.
Anti-integrin receptor antibodies are also well known. Suitable anti-integrin (e.g. αᵥβ₃, αᵥβ₅, αᵥβ₆) monoclonal antibodies can be modified to encompasses antigen binding fragments thereof, including F(ab)₂, Fab, and engineered Fv or single-chain antibody. One suitable and preferably used monoclonal antibody directed against integrin receptor αᵥβ₃ is identified as LM609 (Brooks et al., 1994, Cell 79, 1157; ATCC HB 9537). A potent specific anti-αᵥβ₅ antibody, P1F6, is disclosed in WO 97/45447, which is also preferred according to this invention. A further suitable aᵥβ₆ selective antibody is MAb 14D9.F8 (WO 99/37683, DSM ACC2331, Merck KGaA, Germany) as well as MAb 17.E6 (EP 0719 859, DSM ACC2160, Merck KGaA) which is selectively directed to the αᵥ- chain of integrin receptors. Another suitable anti-integrin antibody is the commercialized Vitraxin ^{®}.

An **"angiogenic growth factor or growth factor receptor"** is a factor or receptor which promotes by its activation the growth and development of blood vessels. Typically, Vascular Endothelial Growth Factor (VEGF) and its receptor belong to this group.

The term **"antibody"** or **"immunoglobulin"** herein is used in the broadest sense and specifically covers intact monoclonal antibodies, polyclonal antibodies, multispecific antibodies (e.g. bispecific antibodies) formed from at least two intact antibodies, and antibody fragments, so long as they exhibit the desired biological activity. The term generally includes heteroantibodies which are composed of two or more antibodies or fragments thereof of different binding specificity which are linked together.

Depending on the amino acid sequence of their constant regions, intact antibodies can be assigned to different **"antibody (immunoglobulin) classes".** There are five major classes of intact antibodies: IgA, IgD, IgE, IgG, and IgM, and several of these may be further divided into "subclasses" (isotypes), e.g., IgG1, IgG2, IgG3, IgG4, IgA, and IgA2. The heavy-chain conscant domains that correspond to the different classes of antibodies are called α, δ, ε, γ and µ respectively. Preferred major class for antibodies according to the invention is IgG, in more detail IgG1 and IgG2.
Antibodies are usually glycoproteins having a molecular weight of about 150,000, composed of two identical light (L) chains and two identical heavy (H) chains. Each light chain is linked to a heavy chain by one covalent disulfide bond, while the number of disulfide linkages varies among the heavy chains of different immunoglobulin isotypes. Each heavy and light chain also has regularly spaced intrachain disulfide bridges. Each heavy chain has at one end a variable domain (VH) followed by a number of constant domains. Each light chain has a variable domain at one end (VL) and a constant domain at its other end. The constant domain of the light chain is aligned with the first constant domain of the heavy chain, and the light-chain variable domain is aligned with the variable domain of the heavy chain. Particular amino acid residues are believed to form an interface between the light chain and heavy chain variable domains. The "light chains" of antibodies from any vertebrate species can be assigned to one of two clearly distinct types, called kappa (κ) and lambda (λ), based on the amino acid sequences of their constant domains.

The term **"monoclonal antibody"** as used herein refers to an antibody obtained from a population of substantially homogeneous antibodies, i.e., the individual antibodies comprising the population are identical except for possible naturally occurring mutations that may be present in minor amounts. Monoclonal antibodies are highly specific, being directed against a single antigenic site. Furthermore, in contrast to polyclonal antibody preparations which include different antibodies directed against different determinants (epitopes), each monoclonal antibody is directed against a single determinant on the antigen. In addition to their specificity, the monoclonal antibodies are advantageous in that they may be synthesized uncontaminated by other antibodies. Methods for making monoclonal antibodies include the hybridoma method described by Kohler and Milstein (1975, Nature 256, 495) and in "Monoclonal Antibody Technology, The Production and Characterization of Rodent and Human Hybridomas" (1985, Burdon et al., Eds, Laboratory Techniques in Biochemistry and Molecular Biology, Volume 13, Elsevier Science Publishers, Amsterdam), or may be made by well known recombinant DNA methods (see, e.g., US 4,816,567). Monoclonal antibodies may also be isolated from phage antibody libraries using the techniques described in Clackson et al., Nature, 352:624-628 (1991) and Marks et al., J. Mol. Biol., 222:58, 1-597(1991), for example.

The term **"chimeric antibody"** means antibodies in which a portion of the heavy and/or light chain is identical with or homologous to corresponding sequences in antibodies derived from a particular species or belonging to a particular antibody class or subclass, while the remainder of the chain(s) is identical with or homologous to corresponding sequences in antibodies derived from another species or belonging to another antibody class or subclass, as well as fragments of such antibodies, so long as they exhibit the desired biological activity (e.g.: US 4,816,567; Morrison et al., Proc. Nat. Acad. Sci. USA, 81:6851-6855 (1984)). Methods for making chimeric and humanized antibodies are also known in the art. For example, methods for making chimeric antibodies include those described in patents by Boss (Celltech) and by Cabilly (Genentech) (US 4,816,397; US 4,816,567).

**"Humanized antibodies"** are forms of non-human (e.g., rodent) chimeric antibodies that contain minimal sequence derived from non-human immunoglobulin. For the most part, humanized antibodies are human immunoglobulins (recipient antibody) in which residues from a hypervariable region (CDRs) of the recipient are replaced by residues from a hypervariable region of a non-human species (donor antibody) such as mouse, rat, rabbit or nonhuman primate having the desired specificity, affinity and capacity. In some instances, framework region (FR) residues of the human immunoglobulin are replaced by corresponding non-human residues. Furthermore, humanized antibodies may comprise residues that are not found in the recipient antibody or in the donor antibody. These modifications are made to further refine antibody performance. In general, the humanized antibody will comprise substantially all of at least one, and typically two, variable domains, in which all or substantially all of the hypervariable loops correspond to those of a non-human immunoglobulin and all or substantially all of the FRs are those of a human immunoglobulin sequence. The humanized antibody optionally also will comprise at least a portion of an immunoglobulin constant region (Fc), typically that of a human immunoglobulin. Methods for making humanized antibodies are described, for example, by Winter (US 5,225,539) and Boss (Celltech, US 4,816,397).

The term "**variable**" or "**FR**" refers to the fact that certain portions of the variable domains differ extensively in sequence among antibodies and are used in the binding and specificity of each particular antibody for its particular antigen. However, the variability is not evenly distributed throughout the variable domains of antibodies. It is concentrated in three segments called hypervariable regions both in the light chain and the heavy chain variable domains. The more highly conserved portions of variable domains are called the framework regions (FRs). The variable domains of native heavy and light chains each comprise four FRs (FR1 - FR4), largely adopting a β-sheet configuration, connected by three hypervariable regions, which form loops connecting, and in some cases forming part of. the β-sheet structure. The hypervariable regions in each chain are held together in close proximity by the FRs and, with the hypervariable regions from the other chain, contribute to the formation of the antigen-binding site of antibodies (see Kabat et al., Sequences of Proteins of Immunological Interest, 5th Ed. Public Health Service, National Institutes of Health, Bethesda, MD. (1991)). The constant domains are not involved directly in binding an antibody to an antigen, but exhibit various effector functions, such as participation of the antibody in antibody dependent cellular cytotoxicity (ADCC).

The term **"hypervariable region"** or **"CDR"** when used herein refers to the amino acid residues of an antibody which are responsible for antigen-binding. The hypervariable region generally comprises amino acid residues from a "complementarity determining region" or "CDR" (e.g. residues 24-34 (L1), 50-56 (L2) and 89-97 (L3) in the light chain variable domain and 31-35 (H1), 50-65 (H2) and 95-102 (H3) in the heavy chain variable domain; and/or those residues from a "hypervariable loop" (e.g. residues 26-32 (L1 ), 50-52 (L2) and 91-96 (L3) in the light chain variable domain and 26-32 (H1), 53-55 (H2) and 96-101 (H3) in the heavy chain variable domain; Chothia and Lesk J. Mol. Biol. 196:901-917 (1987)).

**"Framework Region"** or **"FR"** residues are those variable domain residues other than the hypervariable region residues as herein defined.

**"Antibody fragments"** comprise a portion of an intact antibody, preferably comprising the antigen-binding or variable region thereof. Examples of antibody fragments include Fab, Fab', F(ab')2, Fv and Fc fragments, diabodies, linear antibodies, single-chain antibody molecules; and multispecific antibodies formed from antibody fragment(s). An "intact" antibody is one which comprises an antigen-binding variable region as well as a light chain constant domain (CL) and heavy chain constant domains, CH1, CH2 and CH3. Preferably, the intact antibody has one or more effector functions.
Papain digestion of antibodies produces two identical antigen-binding fragments, called **"Fab"** fragments, each comprising a single antigen-binding site and a CL and a CH1 region, and a residual **"Fc"** fragment, whose name reflects its ability to crystallize readily.
The **"Fc"** region of the antibodies comprises, as a rule, a CH2, CH3 and the hinge region of an IgG1 or IgG2 antibody major class. The hinge region is a group of about 15 amino acid residues which combine the CH1 region with the CH2-CH3 region.
Pepsin treatment yields an **"F(ab')2"** fragment that has two antigen-binding sites and is still capable of cross-linking antigen. **"Fv"** is the minimum antibody fragment which contains a complete antigen-recognition and antigen-binding site. This region consists of a dimer of one heavy chain and one light chain variable domain in tight, non-covalent association. It is in this configuration that the three hypervariable regions (CDRs) of each variable domain interact to define an antigen-binding site on the surface of the VH - VL dimer. Collectively, the six hypervariable regions confer antigen-binding specificity to the antibody. However, even a single variable domain (or half of an Fv comprising only three hypervariable regions specific for an antigen) has the ability to recognize and bind antigen, although at a lower affinity than the entire binding site. The **Fab** fragment also contains the constant domain of the light chain and the first constant domain (CH1) of the heavy chain. "**Fab**'" fragments differ from Fab fragments by the addition of a few residues at the carboxy terminus of the heavy chain CH1 domain including one or more cysteines from the antibody hinge region. F(ab')2 antibody fragments originally were produced as pairs of Fab' fragments which have hinge cysteines between them. Other chemical couplings of antibody fragments are also known (see e.g. Hermanson, Bioconjugate Techniques, Academic Press, 1996; . US 4,342,566). **"Single-chain Fv"** or **"scFv"** antibody fragments comprise the V, and V, domains of antibody, wherein these domains are present in a Single polypeptide chain. Preferably, the Fv polypeptide further comprises a polypeptide linker between the VH and VL domains which enables the scFv to form the desired structure for antigen binding. Single-chain FV antibodies are known, for example, from Plückthun (The Pharmacology of Monoclonal Antibodies, Vol. 113, Rosenburg and Moore eds., Springer-Verlag, New York, pp. 269-315 (1994)), WO93/16185; US 5,571,894; US 5,587,458; Huston et al. (1988, Proc.Natl. Acad. Sci. 85, 5879) or Skerra and Plueckthun (1988, Science 240, 1038).

The term "**diabodies**" refers to small antibody fragments with two antigen-binding sites, which fragments comprise a variable heavy domain (V,) connected to a variable light domain (V,) in the same polypeptide chain (V, - V,). By using a linker that is too short to allow pairing between the two domains on the same chain, the domains are forced to pair with the complementary domains of another chain and create two antigen-binding sites. Diabodies are described more fully in, for example, EP 404,097; WO 93/11161.

**"Bispecific antibodies"** are single, divalent antibodies (or immunotherapeutically effective fragments thereof) which have two differently specific antigen binding sites. For example the first antigen binding site is directed to an angiogenesis receptor (e.g. integrin or VEGF receptor), whereas the second antigen binding site is directed to an ErbB receptor (e.g. EGFR or HER2). Bispecific antibodies can be produced by chemical techniques (see e.g., Kranz et al. (1981) Proc. Natl. Acad. Sci. USA 78, 5807), by "polydoma" techniques (See US 4,474,893) or by recombinant DNA techniques, which all are known per se. Further methods are described in WO 91/00360, WO 92/05793 and WO 96/04305. Bispecific antibodies can also be prepared from single chain antibodies (see e.g., Huston et al. (1988) Proc. Natl. Acad. Sci. 85, 5879; Skerra and Plueckthun (1988) Science 240, 1038). These are analogues of antibody variable regions produced as a single polypeptide chain. To form the bispecific binding agent, the single chain antibodies may be coupled together chemically or by genetic engineering methods known in the art. It is also possible to produce bispecific antibodies according to this invention by using leucine zipper sequences. The sequences employed are derived from the leucine zipper regions of the transcription factors Fos and Jun (Landschulz et al., 1988, Science 240,1759; for review, see Maniatis and Abel, 1989, Nature 341, 24). Leucine zippers are specific amino acid sequences about 20-40 residues long with leucine typically occurring at every seventh residue. Such zipper sequences form amphipathic α-helices, with the leucine residues lined up on the hydrophobic side for dimer formation. Peptides corresponding to the leucine zippers of the Fos and Jun proteins form heterodimers preferentially (O'Shea et al., 1989, Science 245, 646). Zipper containing bispecific antibodies and methods for making them are also disclosed in WO 92/10209 and WO 93/11162. A bispecific antibody according the invention may be an antibody, directed to VEGF receptor and αVβ3 receptor as discussed above with respect to the antibodies having single specificity.

The term **"immunoconjugate"** refers to an antibody or immunoglobulin, respectively, or a immunologically effective fragment thereof, which is fused by covalent linkage to a non-immunologically effective molecule. Preferably this fusion partner is a peptide or a protein, which may be glycosylated. Said non-antibody molecule can be linked to the C-terminal of the constant heavy chains of the antibody or to the N-terminals of the variable light and/or heavy chains. The fusion partners can be linked via a linker molecule, which is, as a rule, a 3 - 15 amino acid residues containing peptide. Immunoconjugates according to the invention comprise preferably fusion proteins consisting of an immunoglobulin or immunotherapeutically effective fragment thereof, directed to an angiogenic receptor, preferably an integrin or VEGF receptor and TNFα or a fusion protein consisting essentially of TNFα and IFNγ or another suitable cytokine, which is linked with its N-terminal to the C-terminal of said immunoglobulin, preferably the Fc portion thereof.

The term **"fusion protein"** refers to a natural or synthetic molecule consisting of one ore more non-immunotherapeutically effective (non-antibody) proteins or peptides having different specificity which are fused together optionally by a linker molecule. Fusion protein according to the invention may be molecules consisting of, for example, cyclo-(Arg-Gly-Asp-DPhe-NMeVal) fused to TNFα and / or IFNγ.

**"Heteroantibodies"** are two or more antibodies or antibody-binding fragments which are linked together, each of them having a different binding specificity. Heteroantibodies can be prepared by conjugating together two or more antibodies or antibody fragments. Preferred heteroantibodies are comprised of cross-linked Fab/Fab' fragments. A variety of coupling or crosslinking agents can be used to conjugate the antibodies. Examples are protein A, carboiimide, N-succinimidyl-S-acetyl-thioacetate (SATA) and N-succinimidyl-3-(2-pyridyldithio) propionate (SPDP) (see e.g., Karpovsky et al. (1984) J. EXP. Med. 160,1686; Liu et a. (1985) Proc. Natl. Acad. Sci. USA 82, 8648). Other methods include those described by Paulus, Behring Inst. Mitt., No. 78, 118 (1985); Brennan et a. (1985) Science 30 m:81 or Glennie et al. (1987) J. Immunol. 139, 2367. Another method uses o-phenylenedimaleimide (oPDM) for coupling three Fab' fragments (WO 91/03493). Multispecific antibodies are in context of this invention also suitable and can be prepared, for example according to the teaching of WO 94/13804 and WO 98/50431.

Antibody **"effector functions"** refer to those biological activities attributable to the Fc region (a native sequence Fc region or amino acid sequence variant Fc region) of an antibody. Examples of antibody effector functions include complement dependent cytotoxicity, Fc receptor binding, antibody-dependent cell-mediated cytotoxicity (ADCC), phagocytosis; down regulation of cell surface receptors (e.,g. B cell receptor), etc.

The term **"ADCC"** (antibody-dependent cell-mediated cytotoxicity) refers to a cell-mediated reaction in which nonspecific cytotoxic cells that express Fc receptors (FcR) (e.g. natural killer (NK) cells, neutrophils, and macrophages) recognize bound antibody on a target cell and subsequently cause lysis of the target cell. The primary cells for mediating ADCC, NK cells, express FcγRIII only, whereas monocytes express FcγRI, FcγRII and FcγRIII. To assess ADCC activity of a molecule of interest, an *in vitro* ADCC assay, such as that described in the prior art (US 5,500,362; US 5,821,337) may be performed. Useful effector cells for such assays include peripheral blood mononuclear cells (PBMC) and natural killer (NK) cells.
**"Human effector cells"** are leukocytes which express one or more FcRs and perform effector functions. Preferably, the cells express at least FcγRIII and perform ADCC effector function. Examples of human leukocytes which mediate ADCC include peripheral blood mononuclear cells (PBMC), natural killer (NK) cells, monocytes, cytotoxic T cells and neutrophils.

The terms **"Fc receptor"** or "FcR" are used to describe a receptor that binds to the Fc region of an antibody. The preferred FcR is a native sequence human FcR. Moreover, a preferred FcR is one which binds an IgG antibody (a gamma receptor) and includes receptors of the FcγRI, FcγRII, and FcγRIII subclasses, including allelic variants and alternatively spliced forms of these receptors. FcRs are reviewed, for example, in Ravetch and Kinet, Annu. Rev. Immunol 9:457-92 (1991).

The term **"cytokine"** is a generic term for proteins released by one cell population which act on another cell as intercellular mediators. Examples of such cytokines are lymphokines, monokines, and traditional polypeptide hormones. Included among the cytokines are growth hormone such as human growth hormone, N-methionyl human growth hormone, and bovine growth hormone; parathyroid hormone; thyroxine; insulin; proinsulin; relaxin; prorelaxin; glycoprotein hormones such as follicle stimulating hormone (FSH), thyroid stimulating hormone (TSH), and luteinizing hormone (LH); hepatic growth factor; fibroblast growth factor; prolactin; placental lactogen; mouse gonadotropin-associated peptide; inhibin; activin; vascular endothelial growth factor (VEGF); integrin; thrombopoietin (TPO); nerve growth factors such as NGFβ; platelet-growth factor; transforming growth factors (TGFs) such as TGFα and TGFβ; erythropoietin (EPO); interferons such as IFNα, IFNβ, and IFNγ; colony stimulating factors such as M-CSF, GM-CSF and G-CSF; interleukins such as IL-1, IL-1a, IL-2, IL-3, IL-4, IL-5, IL-6, IL-7, IL-8, IL-9, IL-10, IL-11, IL-12; and TNFα or TNFβ. Preferred cytokines according to the invention are interferons and TNFa.

The term **"cytotoxic agent"** as used herein refers to a substance that inhibits or prevents the function of cells and/or causes destruction of cells. The term is intended to include radioactive isotopes, chemotherapeutic agents, and toxins such as enzymatically active toxins of bacterial, fungal, plant or animal origin, or fragments thereof. The term may include also members of the cytokine family, preferably IFNγ.

The term **"chemotherapeutic agent"** or **"anti-neoplastic agent"** includes chemical agents that exert anti-neoplastic effects, i.e., prevent the development, maturation, or spread of neoplastic cells, directly on the tumor cell, e.g., by cytostatic or cytotoxic effects, and not indirectly through mechanisms such as biological response modification. Suitable chemotherapeutic agents according to the invention are preferably natural or synthetic chemical compounds, but biological molecules, such as proteins, polypeptides etc. are not expressively excluded. There are large numbers of anti-neoplastic agents available in commercial use, in clinical evaluation and in pre-clinical development, which could be included in the present invention for treatment of tumors / neoplasia by combination therapy with TNFα and the anti-angiogenic agents as cited above, optionally with other agents such as EGF receptor antagonists. It should be pointed out that the chemotherapeutic agents can be administered optionally together with above-said drug combination.
Examples of chemotherapeutic or agents include alkylating agents, for example, nitrogen mustards, ethyleneimine compounds, alkyl sulphonates and other compounds with an alkylating action such as nitrosoureas, cisplatin and dacarbazine; antimetabolites, for example, folic acid, purine or pyrimidine antagonists; mitotic inhibitors, for example, vinca alkaloids and derivatives of podophyllotoxin; cytotoxic antibiotics and camptothecin derivatives. Preferred chemotherapeutic agents or chemotherapy include amifostine (ethyol), cisplatin, dacarbazine (DTIC), dactinomycin, mechlorethamine (nitrogen mustard), streptozocin, cyclophosphamide, carrnustine (BCNU), lomustine (CCNU), doxorubicin (adriamycin), doxorubicin lipo (doxil), gemcitabine (gemzar), daunorubicin, daunorubicin lipo (daunoxome), procarbazine, mitomycin, cytarabine, etoposide, methotrexate, 5-fluorouracil (5-FU), vinblastine, vincristine, bleomycin, paclitaxel (taxol), docetaxel (taxotere), aldesleukin, asparaginase, busulfan, carboplatin, cladribine, camptothecin, CPT-11, 1 0-hydroxy-7-ethyl-camptothecin (SN38), dacarbazine, floxuridine, fludarabine, hydroxyurea, ifosfamide, idarubicin, mesna, interferon alpha, interferon beta, irinotecan, mitoxantrone, topotecan, leuprolide, megestrol, melphalan, mercaptopurine, plicamycin, mitotane, pegaspargase, pentostatin, pipobroman, plicamycin, streptozocin, tamoxifen, teniposide, testolactone, thioguanine, thiotepa,. uracil mustard, vinorelbine, chlorambucil and combinations thereof.
Most preferred chemotherapeutic agents according to the invention are cisplatin, gemcitabine, doxorubicin, paclitaxel (taxol) and bleomycin.

The terms **"cancer" and "tumor"** refer to or describe the physiological condition in mammals that is typically characterized by unregulated cell growth. By means of the pharmaceutical compositions according of the present invention tumors can be treated such as tumors of the breast, heart, lung, small intestine, colon, spleen, kidney, bladder, head and neck, ovary, prostate, brain, pancreas, skin, bone, bone marrow, blood, thymus, uterus, testicles, cervix, and liver. More specifically the tumor is selected from the group consisting of adenoma, angio-sarcoma, astrocytoma, epithelial carcinoma, germinoma, glioblastoma, glioma, hamartoma, hemangioendothelioma, hemangiosarcoma, hematoma, hepato-blastoma, leukemia, lymphoma, medulloblastoma, melanoma, neuroblastoma, osteosarcoma, retinoblastoma, rhabdomyosarcoma, sarcoma and teratoma. In detail, the tumor is selected from the group consisting of acral lentiginous melanoma, actinic keratoses, adenocarcinoma, adenoid cycstic carcinoma, adenomas, adenosarcoma, adenosquamous carcinoma, astrocytic tumors, bartholin gland carcinoma, basal cell carcinoma, bronchial gland carcinomas, capillary, carcinoids, carcinoma, carcinosarcoma, cavernous, cholangiocarcinoma, chondosarcoma, choriod plexus papilloma/carcinoma, clear cell carcinoma, cystadenoma, endodermal sinus tumor, endometrial hyperplasia, endometrial stromal sarcoma, endometrioid adenocarcinoma, ependymal, epitheloid, Ewing's sarcoma, fibrolamellar, focal nodular hyperplasia, gastrinoma, germ cell tumors, glioblastoma, glucagonoma, hemangiblastomas, hemangioendothelioma, hemangiomas, hepatic adenoma, hepatic adenomatosis, hepatocellular carcinoma, insulinoma, intaepithelial neoplasia, interepithelial squamous cell neoplasia, invasive squamous cell carcinoma, large cell carcinoma, leiomyosarcoma, lentigo maligna melanomas, malignant melanoma, malignant mesothelial tumors, medulloblastoma, medulloepithelioma, melanoma, meningeal, mesothelial, metastatic carcinoma, mucoepidermoid carcinoma, neuroblastoma, neuroepithelial adenocarcinoma nodular melanoma, oat cell carcinoma, oligodendroglial, osteosarcoma, pancreatic polypeptide, papillary serous adeno-carcinoma, pineal cell, pituitary tumors, plasmacytoma, pseudo-sarcoma, pulmonary blastoma, renal cell carcinoma, retinoblastoma, rhabdomyo-sarcoma, sarcoma, serous carcinoma, small cell carcinoma, soft tissue carcinomas, somatostatin-secreting tumor, squamous carcinoma, squamous cell carcinoma, submesothelial, superficial spreading melanoma, undifferentiated carcinoma, uveal melanoma, verrucous carcinoma, vipoma, well differentiated carcinoma, and Wilm's tumor.

An **"ErbB receptor"** is a receptor protein tyrosine kinase which belongs to the ErbB receptor family and includes EGFR(ErbB1), ErbB2, ErbB3 and ErbB4 receptors and other members of this family to be identified in the future. The ErbB receptor will generally comprise an extracellular domain, which may bind an ErbB ligand; a lipophilic transmembrane domain; a conserved intracellular tyrosine kinase domain; and a carboxyl-terminal signaling domain harboring several tyrosine residues which can be phosphorylated. The ErbB receptor may be a "native sequence" ErbB receptor or an "amino acid sequence variant" thereof. Preferably the ErbB receptor is native sequence human ErbB receptor. ErbB1 refers to the gene encoding the EGFR protein product. Mostly preferred is the EGF receptor (HER1). The expressions "ErbB1" and "HER1" are used interchangeably herein and refer to human HER1 protein. The expressions "ErbB2" and "HER2" are used interchangeably herein and refer to human HER2 protein. ErbB1 receptors (EGFR) are preferred according to this invention

**"ErbB ligand"** is a polypeptide which binds to and/or activates an ErbB receptor. ErbB ligands which bind EGFR include EGF, TGF-a, amphiregulin, betacellulin, HB-EGF and epiregulin.

The term **"ErbB receptor antagonist / inhibitor"** refers to a natural or synthetic molecule which binds and blocks or inhibits the ErbB receptor. Thus, by blocking the receptor the antagonist prevents binding of the ErbB ligand (agonist) and activation of the agonist/ligand receptor complex. ErbB antagonists may be directed to HER1 (EGFR) or HER2. Preferred antagonists of the invention are directed to the EGF receptor (EGFR, HER1). The ErbB receptor antagonist may be an antibody or an immunotherapeutically effective fragment thereof or non-immunobiological molecules, such as a peptide, polypeptide protein. Chemical molecules are also included, however, anti-EGFR antibodies and anti-HER2 antibodies are the preferred antagonists according to the invention.
Preferred antibodies of the invention are anti-Her1 and anti-Her2 antibodies, more preferably anti-Her1 antibodies. Preferred anti-Her1 antibodies are MAb 425, preferably humanized MAb 425 (hMAb 425, US 5,558,864; EP 0531 472) and chimeric MAb 225 (cMAb 225, US 4,943,533 and EP 0359 282). Most preferred is monoclonal antibody h425, which has shown in mono-drug therapy high efficacy combined with reduced adverse and side effects. Most preferred anti-HER2 antibody is HERCEPTIN^{®} commercialized by Genentech/Roche.
Efficacious EGF receptor antagonists according to the invention may be also natural or synthetic chemical compounds. Some examples of preferred molecules of this category include organic compounds, organometallic compounds, salts of organic and organometallic compounds.
Examples for HER2 receptor antagonists are: styryl substituted heteroaryl compounds (US 5,656,655); bis mono and/or bicyclic aryl heteroaryl, carbocyclic, and heterocarbocyclic compounds (US 5,646,153); tricyclic pyrimidine compounds (US 5,679,683); quinazoline derivatives having receptor tyrosine kinase inhibitory activity (US 5,616,582); heteroarylethenediyl or heteroaryl-ethenediylaryl compounds (US 5,196,446); a compound designated as 6-(2,6-dichlorophenyl)-2-(4-(2-diethyl-aminoethoxy) phenylamino)-8-methyl-8H-pyrido(2,3)-5-pyrimidin-7-one (Panek, et al., 1997, J. Pharmacol. Exp. Therap. 283,1433) inhibiting EGFR, PDGFR, and FGFR families of receptors.

**"Radiotherapy":** The tumors which can be treated with the pharmaceutical compositions according to the invention can additionally be treated with radiation or radiopharmaceuticals .The source of radiation can be either external or internal to the patient being treated. When the source is external to the patient, the therapy is known as external beam radiation therapy (EBRT). When the source of radiation is internal to the patient, the treatment is called brachytherapy (BT). Some typical radioactive atoms that have been used include radium, cesium-137, and iridium-192, americium-241 and gold-198, Cobalt-57; Copper-67; Technetium-99; lodide-123; lodide-131; and Indium-111. It is also possible to label the agents according to the invention with radioactive isotopes.
Today radiation therapy is the standard treatment to control unresectable or inoperable tumors and / or tumor metastases. Improved results have been seen when radiation therapy has been combined with chemotherapy. Radiation therapy is based on the principle that high-dose radiation delivered to a target area will result in the death of reproductive cells in both tumor and normal tissues. The radiation dosage regimen is generally defined in terms of radiation absorbed dose (rad) , time and fractionation, and must be carefully defined by the oncologist. The amount of radiation a patient receives will depend on various consideration but the two most important considerations are the location of the tumor in relation to other critical structures or organs of the body, and the extent to which the tumor has spread. A preferred course of treatment for a patient undergoing radiation therapy will be a treatment schedule over a 5 to 6 week period, with a total dose of 50 to 60 Gy administered to the patient in a single daily fraction of 1.8 to 2.0 Gy, 5 days a week. A Gy is an abbreviation for Gray and refers to 100 rad of dose. In the preferred embodiment, there is synergy when tumors in human patients are treated with the angiogenesis antagonist and TNFα/IFNγ and radiation. In other words, the inhibition of tumor growth by means of said compounds is enhanced when combined with radiation and / or chemotherapeutic agents. Radiation therapy can be optionally used according to the invention. It is recommended and preferred in cases in which no sufficient amounts of the agents according to the invention can be administered to the patient.

**"Pharmaceutical treatment":** The method of the invention comprises a variety of modalities for practicing the invention in terms of the steps. For example, the agents according to the invention can be administered simultaneously, sequentially, or separately. Furthermore, the agents can be separately administered within a time interval of about 3 weeks between administrations, i.e., from substantially immediately after the first active agent is administered to up to about 3 weeks after the first agent is administered. The method can be practiced following a surgical procedure. Alternatively, the surgical procedure can be practiced during the interval between administration of the first active agent and the second active agent. Exemplary of this method is the combination of the present method with surgical tumor removal. Treatment according to the method will typically comprise administration of the therapeutic compositions in one or more cycles of administration. For example, where a simultaneous administration is practiced, a therapeutic composition comprising both agents is administered over a time period of from about 2 days to about 3 weeks in a single cycle. Thereafter, the treatment cycle can be repeated as needed according to the judgment of the practicing physician. Similarly, where a sequential application is contemplated, the administration time for each individual therapeutic will be adjusted to typically cover the same time period. The interval between cycles can vary from about zero to 2 months.

The agents of this invention can be administered parenterally by injection or by gradual infusion over time. Although the tissue to be treated can typically be accessed in the body by systemic administration and therefore most often treated by intravenous administration of therapeutic compositions, other tissues and delivery means are contemplated where there is a likelihood that the tissue targeted contains the target molecule. Thus, the agents of this invention can be administered intraocularly, intravenously, intraperitoneally, intramuscularly, subcutaneously, intracavity, transdermally, by orthotopic injection and infusion, and can also be delivered by peristaltic means. The therapeutic compositions containing, for example, an integrin antagonist of this invention are conventionally administered intravenously, as by injection of a unit dose, for example.
Therapeutic compositions of the present invention contain a physiologically tolerable carrier together with the relevant agent as described herein, dissolved or dispersed therein as an active ingredient. As used herein, the term **"pharmaceutically acceptable"** refers to compositions, carriers, diluents and reagents which represent materials that are capable of administration to or upon a mammal without the production of undesirable physiological effects such as nausea, dizziness, gastric upset and the like. The preparation of a pharmacological composition that contains active ingredients dissolved or dispersed therein is well understood in the art and need not be limited based on formulation. Typically, such compositions are prepared as injectables either as liquid solutions or suspensions, however, solid forms suitable for solution, or suspensions, in liquid prior to use can also be prepared. The preparation can also be emulsified. The active ingredient can be mixed with excipients which are pharmaceutically acceptable and compatible with the active ingredient and in amounts suitable for use in the therapeutic methods described herein. Suitable excipients are, for example, water, saline, dextrose, glycerol, ethanol or the like and combinations thereof. In addition, if desired, the composition can contain minor amounts of auxiliary substances such as wetting or emulsifying agents, pH buffering agents and the like which enhance the effectiveness of the active ingredient. The therapeutic composition of the present invention can include pharmaceutically acceptable salts of the components therein. Pharmaceutically acceptable salts include the acid addition salts (formed with the free amino groups of the polypeptide) that are formed with inorganic acids such as. for example, hydrochloric or phosphoric acids, or such organic acids as acetic, tartaric, mandelic and the like. Salts formed with the free carboxyl groups can also be derived from inorganic bases such as, for example, sodium, potassium, ammonium, calcium or ferric hydroxides, and such organic bases as isopropylamine, trimethylamine, 2-ethylamino ethanol, histidine, procaine and the like. Particularly preferred is the HCl salt when used in the preparation of cyclic polypeptide αv antagonists. Physiologically tolerable carriers are well known in the art. Exemplary of liquid carriers are sterile aqueous solutions that contain no materials in addition to the active ingredients and water, or contain a buffer such as sodium phosphate at physiological pH value, physiological saline or both, such as phosphate-buffered saline. Still further, aqueous carriers can contain more than one buffer salt, as well as salts such as sodium and potassium chlorides, dextrose, polyethylene glycol and other solutes. Liquid compositions can also contain liquid phases in addition to and to the exclusion of water. Exemplary of such additional liquid phases are glycerin. vegetable oils such as cottonseed oil, and water-oil emulsions.
Typically, a therapeutically effective amount of an immunotherapeutic agent, for example, in the form of an integrin receptor blocking antibody or antibody fragment or antibody conjugate or an anti-VEGF receptor blocking antibody, fragment or conjugate is an amount such that when administered in physiologically tolerable composition is sufficient to achieve a plasma concentration of from about 0.01 microgram (µg) per milliliter (ml) to about 100 µg/ml, preferably from about 1 µg/ml to about 5 µg/ml and usually about 5 µg/ml. Stated differently. the dosage can vary from about 0.1 mg/kg to about 300 mg/kg, preferably from about 0.2 mg/kg to about 200 mg/kg, most preferably from about 0.5 mg/kg to about 20 mg/kg, in one or more dose administrations daily for one or several days. Where the immunotherapeutic agent is in the form of a fragment of a monoclonal antibody or a conjugate, the amount can readily be adjusted based on the mass of the fragment / conjugate relative to the mass of the whole antibody. A preferred plasma concentration in molarity is from about 2 micromolar (µM) to about 5 millimolar (mM) and preferably, about 100 µM to 1 mM antibody antagonist.
A therapeutically effective amount of an agent according of this invention which is a non-immunotherapeutic peptide or a protein polypeptide (e.g. TNFα, IFNγ), or other similarly-sized biological molecule, is typically an amount of polypeptide such that when administered in a physiologically tolerable composition is sufficient to achieve a plasma concentration of from about 0.1 microgram (µg) per milliliter (ml) to about 200 µg/ml, preferably from about 1 µg/ml to about 150 µg/ml. Based on a polypeptide having a mass of about 500 grams per mole, the preferred plasma concentration in molarity is from about 2 micromolar (µM) to about 5 millimolar (mM) and preferably about 100 µM to 1 mM polypeptide antagonist.
The typical dosage of an active agent, which is a preferably a chemical antagonist or a (chemical) chemotherapeutic agent according to the invention (neither an immunotherapeutic agent nor a non-immunotherapeutic peptide/protein) is 10 mg to 1000 mg, preferably about 20 to 200 mg, and more preferably 50 to 100 mg per kilogram body weight per day.
The pharmaceutical compositions of the invention can comprise phrase encompasses treatment of a subject with agents that reduce or avoid side effects associated with the combination therapy of the present invention ("adjunctive therapy"), including, but not limited to, those agents, for example, that reduce the toxic effect of anticancer drugs, e.g., bone resorption inhibitors, cardioprotective agents. Said adjunctive agents prevent or reduce the incidence of nausea and vomiting associated with chemotherapy, radiotherapy or operation, or reduce the incidence of infection associated with the administration of myelosuppressive anticancer drugs. Adjunctive agents are well known in the art.
The immunotherapeutic agents according to the invention can additionally administered with adjuvants like BCG and immune system stimulators. Furthermore, the compositions may include immunotherapeutic agents or chemotherapeutic agents which contain cytotoxic effective radio labeled isotopes, or other cytotoxic agents, such as a cytotoxic peptides (e.g. cytokines) or cytotoxic drugs and the like.

The term **" pharmaceutical kit"** for treating tumors or tumor metastases refers to a package and, as a rule, instructions for using the reagents in methods to treat tumors and tumor metastases. A reagent in a kit of this invention is typically formulated as a therapeutic composition as described herein, and therefore can be in any of a variety of forms suitable for distribution in a kit. Such forms can include a liquid, powder, tablet, suspension and the like formulation for providing the antagonist and/or the fusion protein of the present invention. The reagents may be provided in separate containers suitable for administration separately according to the present methods, or alternatively may be provided combined in a composition in a single container in the package. The package may contain an amount sufficient for one or more dosages of reagents according to the treatment methods described herein. A kit of this invention also contains "instruction for use" of the materials contained in the package.

### DESCRIPTION OF THE FIGURES

**Figure 1.** HUVEC spheroid formation and survival does not require integrin ligation. **(*a*)** A blocking anti-VE-cadherin (75) mAb or Ca²⁺-depletion (EDTA, EDTA/Ca²⁺) inhibited HUVEC spheroid formation, while blocking mAbs against integrin α1 (Lia1/2), α5 (SAM-1), αVβ3 (LM609) and PECAM-1 (10D9) or a RGD peptide did not. **(*b*)** Viability. HUVEC recovered from spheroids (○) or fibronectin (●) cultures had similar viability profiles.
**Figure 2.** Integrin-dependent adhesion protects HUVEC against TNF-induced apoptosis. **(*a*)** YoPro-1 uptake: exposure to TNF (T) and TNF/IFNγ (TI) did not induce YoPro-1 staining in fibronectin-adherent HUVEC while it caused a strong YoPro-1 staining in HUVEC spheroids, which was suppressed by the caspase inhibitors BOC and ZVAD. TNF±IFNγ (TI). C, untreated cultures. **(*b*)** Demonstration of caspase-3 activation and PARP cleavage (arrowheads) by Western blotting in TNF/IFNγ-treated (TI) spheroids but not in fibronectin-adherent HUVEC. C, untreated cultures. **(*c*, *d*)** Viability curves of HUVEC exposed to TNF (■), TNF/IFNγ (▲) or control medium (○). **(*e*)** Viability of HUVEC cultured on immobilized antibodies (imAbs) directed against α1 (△/▲), αVβ3 (□/■) and α4 integrins (○/●) in the absence (open symbols) or presence (closed symbols) of TNF/IFNγ
**Figure 3.** TNF-induced NF-κB activation does not require integrin ligation **(*a*)** Western blotting and **(*b*)** electrophoretic mobility shift assays (EMSA) demonstrate paralleling kinetics of I-κB phosphorylation (Pi-κB), I-κB degradation (I-κB) and NF-κB nuclear translocation (EMSA) in fibronectin-adherent HUVEC and spheroids exposed to TNF/IFNγ. **(*c*)** Flow cytometry analysis showing identical induction of ICAM-1 cell surface expression on fibronectin and spheroid HUVEC cultures exposed to TNF (-----) or TNF/IFNγ ). (.....) untreated cells. PECAM-1 expression is shown as contrõl
**Figure 4.** Activation of Akt is essential for HUVEC survival and requires integrin ligation. **(*a*)** Detection of phosphorylated (Pi-Akt) and total Akt (Akt) in fibronectin-adherent and spheroid HUVEC cultures stimulated with TNF/IFNγ for the indicated time. **(*b*)** Left panel: the PI-3 kinase inhibitors wortmannin (W) and LY294002 (LY) sensitized fibronectin-adherent HUVEC to TNF (T) and TNF/IFNγ (TI) -induced apoptosis. Dead cells were visualized by YoPro-1 staining. Right panel: survival curve of fibronectin-adherent HUVEC exposed to LY294002 (●), TNF/IFNγ(Δ), or LY294002/TNF/IFNγ(▲). (○) untreated cultures. **(*c*)** Constitutive active PI-3 kinase (p110*) and Akt (Aktmp), but no wild type Akt (Aktwt) or control plasmid (pBS), promoted survival of spheroid exposed to TNF (●) or TNF/IFN□▲). (○) untreated cultures̃ Inserts represent flow cytometry analysis of EGFP fluorescence of transfected cells (% positive cells). **(*d*)** HUVEC electroporated with control plasmid (pBS) or constitutive active Akt (Akt*) and infected with AdΔNI-κB or AdLacZ were cultured as fibronectin-adherent monolayer or spheroids in the absence (C) or in the presence of TNF (T) or TNF/IFNγ (TI). Apoptotic cells were detected by YoPro-1 staining. Viable fibronectin-adherent cells were stained by crystal violet. **(*e*)** HUVEC electroporated with control plasmid (open symbols) or pAktmp (closed symbols) and infected with AdΔNI-κB (○/•) or AdLacZ (△/▲) and were cultured on fibronectin in the presence of graded concentrations of TNF and viable attached cells were determined by measuring the O.D. of crystal violet-stained wells. **(*f*)** Flow cytometry analysis of ICAM-1 expression in untreated HUVEC (.....), or HUVEC treated with TNF (-----) and TNF/LY294002 (-) (left panel), as well as HUVEC infected with ADANI-κB (middle panel) or AdLacZ and exposed to TNF (-----) and TNF/IFNγ (-).
**Figure 5.** (a-c) Western blotting analysis of Pi-Akt, MDM2, p53, Pi-FKHR/FRKHL1 (a), and Pi-MEK, Pi-p38 and Pi-JNK and Pi-ERK in fibronectin and spheroid HUVEC cultures exposed to TNF/IFNγ for the indicated time. Total Akt, FKHR, MEK, p38, ERK, and JNK protein are shown to demonstrate equal total protein. Spheroid cultures have deficient phosphorylation of Akt and FKHR/FKRL1, increased levels of p53 and enhanced phosphorylation of MEK, p38, ERK and JNK in response to TNF/IFNγ compared to fibronectin-adherent cells.
**Figure 6.** Decreased integrin ligation enhances TNF cytotoxicity in vitro. **(*a*)** HUVEC were cultured on fibronectin or PLL for 16 hours in the absence (C) or presence of TNF (T) or TNF/IFNγ (TI). Apoptotic and viable, adherent cells were revealed by YoPro-1 and crystal violet staining, respectively. **(*b*)** EMD121974 disrupted αVβ3-mediated HUVEC adhesion on gelatin (■) but not the α5β1 component of the α5β1/αVβ3-mediated adhesion to fibronectin (●). The control peptide EMD135981 was ineffective (open symbols). **(*c*)** HUVEC were cultured on fibronectin in the absence (C) or presence of TNF/IFNγ (TI), EMD121974 and EMD135981 as indicated. Apoptotic and adherent cells were revealed by YoPro-1staining and contrast microscopy, respectively. **(*d*)** Viability curves of HUVEC of experiment in panel c. No peptide (○/●); EMD121974 (△/▲); EMD135981 (□/■). Untreated cultures, open symbols. TNF/IFNγ-treated cultures, closed symbols. **(*e*)** Viability curves of HUVEC electroporated with Aktmp (open symbols) or pBS (closed symbols), and cultured on fibronectin and exposed to TNF/IFN□ alone (○/●) or in the presence of EMD121974 (△/▲) or EMD135981 (□/■) peptides. Aktmp prevented cell death induced by combined EMD121974 and TNF/IFN□treatment.
**Figure 7.** Decreased integrin ligation enhances TNF cytotoxicity in in vivo. BN rats bearing the BN-175 syngeneic soft tissue sarcoma were treated with EMD121974 (□), TNF (△) or EMD121974/TNF (■) by ILP technique. (○) sham-treated rats. Tumor growth was measured for 6 days after ILP. Results represent the mean tumor volume ± s.e.m. (n=6). Small fragments of the syngeneic soft tissue sarcoma BN-175 were implanted in the right hind limb of male BN rats, and treatment started when diameter reached 12-14 mm (Manusama et al., Oncol. Rep. 6, 173-177. (1999)). The femoral artery and vein were canulated with silastic tubing and collaterals occluded with a tourniquet. The perfusion was performed for 30 min with 5 ml Heamaccel® (2.4 ml/min) in which the drugs were added as boluses (EMD121974, 500 µg, end concentration in perfusate 170 µM; TNF, 50 µg). The perfusate was oxygenated and the leg kept on 38-39°C with a warm mattress. Rats perfused with EMD121974 also received systemic administration of the peptide 2 hours before and 3 hours after ILP (100 mg/kg i.p.). Tumor diameter was measured in two directions by caliper measurements and tumor volume (V) was calculated (V = 0.4)(A²XB, where B represents the largest diameter and A the diameter perpendicular to B). 6 rats were treated per group. Local and systemic side effects were evaluated as described (Manusama et al., Oncol. Rep. 6, 173-177. (1999)).
**Figure 8.** Decreased integrin ligation enhances TNF-, TRAIL- and FasL-induced cytotoxicity in vitro. HUVEC were cultured overnight on fibronectin coated microtiter plates in the absence (control) or presence of EMD121974 (300 µM), TNF (200 ng/ml), FasL (200 ng/ml), TRAIL (200 ng/ml), LIGHT (200 ng/ml), and IFNγ (330 ng/ml) as indicated. Viability was detemined by MST assays.

The invention can be described in more detail by the following Examples:

### Example 1: Integrin-dependent adhesion endothelial cells against TNFα - induced apoptosis.

### HUVEC spheroid formation and survival does not require integrins

To test the effect of integrin ligation on TNF-induced apoptosis we identified conditions where endothelial cells could be cultured without integrin-dependent adhesion. Single cell suspensions of endothelial cells rapidly die by anoikis (Meredith et al., Mol. Biol Cell. 4, 953-961 (1993)) thus precluding further analysis. But by seeding human umbilical vein endothelial cells (HUVEC) at high density (1.0x106 cells/ml) in BSA-coated wells multicellular spheroids formed within 2-4 hours, and could be maintained for over 24 hours dependent on VE-cadherin and without any detectable contribution from integrins. Inhibition of VE-cadherin activity by blocking monoclonal antibody (mAb), or by depletion of Ca²⁺ - Mg²⁺ with EDTA, blocked spheroid formation, while inhibitory mAbs against α2, α3, α5, α5, β1, αVβ3 or αVβ5 integrins, RGD-based blocking peptides and a blocking anti-PECAM-1 mAb, alone or in combination, did not affect the HUVEC spheroids (Fig. 1 a, and data not shown).
To determine the effect of spheroid culture on cell viability, spheroids and fibronectin-adherent HUVEC were recovered between 6 and 72 hours after plating, serially diluted and further cultured for an additional 48 hours before relative cell number was determined. A shift-to-the left or a flattening of the dilution curve indicates decreased viability. At 6, 12, 16 and 24 hours after plating the viability of HUVEC recovered from spheroid cultures was comparable to that of fibronectin-adherent cultures, but from 36 hours it progressively decreased (Fig. 1 b at 16 hours, and data not shown).
Taken together these results demonstrate that HUVEC can form spheroids and are viable for over 24 hours in the absence of integrin-dependent adhesion.

### Example 2: Adhesion to fibronectin protects HUVEC against TNF-induced apoptosis.

To test whether integrins modulate TNF-induced apoptosis, we cultured HUVEC on fibronectin (integrin-dependent adhesion) or as spheroids (integrin-independent adhesion) in the absence or presence of TNF (200 ng/ml) and of IFNγ (330 ng/ml), an enhancer of TNF cytotoxicity (Dealtry et al., Eur. J. Immunol. 17, 689-693 (1987)). Exposure of monolayers of HUVECs on fibronectin ("fibronectin-adherent HUVEC") to TNF±IFNγ did not increase apoptosis as demonstrated by the absence of YoPro-1 uptake (Idziorek et al., J. Immunol. Methods 185, 249-258 (1995)), cell surface-binding of annexin V, activation of caspase-3 and cleavage of its substrate PARP (Fig. 2a, 2b and data not shown). In contrast, spheroids treated with TNF±IFNγ increased uptake of YoPro-1 (an increase suppressed by caspase inhibitors BOC, Z-VAD, IETD and DVED), DNA fragmentation, caspase-3 activation and cleavage of PARP (Fig. *2a, 2b* and data not shown). To examine the effect of TNF±IFNγ on cell survival we determined the viability of untreated and treated cultures. Exposure of fibronectin-adherent HUVEC to TNF±IFNγ had no effect on cell viability (Fig. 2*c*). Treatment of spheroids with TNF resulted in over 80% cell death and combined TNF/IFNγ treatment caused complete cell death (Fig. 2*d*). Treatment with IFNγ alone was not cytotoxic (data not shown). HUVEC adhere to immobilized fibronectin via αVβ1 and α5β1 integrins (Rüegg et al., Nature Med. 4, 408-414 (1998)). To test for the individual contribution of these integrins to cell survival on fibronectin, we cultured HUVEC on plastic-immobilized mAbs (imAbs) directed against αVβ3, α1, α5 and α4 integrins.
Immobilized anti-αVβ3, anti-α5 and anti-α1 mAbs protected HUVEC against TNF-induced death while anti-α4 mAbs did not (Fig. 2*e* and data not shown). From these results we concluded that αVβ3 and αVβ1 integrin-mediated adhesion suppresses TNF-induced apoptosis, and its lack sensitizes HUVEC to TNF and caspase-mediated apoptosis.

### Example 2: Integrin-dependent signaling protects endothelial cells against TNFα -induced apoptosis.

### TNF-induced NF-κB activation does not require integrin ligation

Activation of the nuclear factor-κB (NF-κB) promotes survival of cells exposed to TNF (Beg & Baltimore, Science 274, 782-784; Van Antwerp et al., Science 274, 787-789 (1996)). Since cell adhesion via integrins activates NF-κB (Scatena et al., J. Cell Biol. 141, 1083-1093 (1998)), we investigated whether the sensitivity of spheroids to TNF-induced apoptosis was due to lack of NF-κB activation. NF-κB activation was assessed by measuring I-κB phosphorylation and degradation, NF-κB nuclear translocation and cell surface expression of ICAM-1, an NF-κB-induced gene (Collins et al., Faseb J. 9, 899-909. (1995)), in spheroid and fibronectin-adherent HUVEC cultures exposed to TNF±IFNγ. We did not observe significant differences in I-κB phosphorylation and degradation, NF-κB nuclear translocation or ICAM-1 expression (Fig. 3a-c), indicating that TNF-induced apoptosis of HUVEC cultured in spheroids was not due to impaired NF-κB activation.

### Example 3:Activation depends on integrin ligation and is essential for cell survival

Next, the activation of Akt / PKB was analyzed, a protein kinase activated by TNF that promotes endothelial cell survival (Madge & Pober, J. Biol. Chem. 275, 15458-15465. (2000)). A basal Akt phosphorylation in fibronectin-adherent HUVEC was increased by exposure to TNF/IFNγ, consistent with a constitutive and a TNF-induced Akt activation. In contrast, no Akt phosphorylation was observed in untreated spheroids, and exposure to TNF/IFNγ induced only a weak phosphorylation (Fig. 4*a*). To assess the relevance of Akt activation to HUVEC survival, we treated fibronectin-adherent cells with wortmannin and LY294002, two pharmacological inhibitors of phosphoinositide-3 (PI-3) kinase, an upstream activator of Akt (Kandel, & Hay, Exp. Cell Res. 253, 210-229. (1999)). We also expressed a constitutively active form of Akt (Aktmp) and PI-3 kinase catalytic subunit (p110*) in spheroids. Wortmannin and LY294002 treatment caused increased apoptosis and decreased survival of fibronectin-adherent cells exposed to TNF±IFNγ (Fig. 4*b*), while Aktmp and p110*, but not wild type Akt (Aktwt) or a control plasmid (pBS), protected spheroids from TNF±IFNγ-induced apoptosis (Fig. 4*c*).
From these results we concluded that activation of Akt was essential for the survival of HUVEC exposed to TNF±IFNγ, and that both basal and TNF-induced Akt activation depended on integrin ligation.

### Example 4: Survival of TNF-treated HUVEC requires activation of Akt and NF-κB

Aktmp suppresses TNF-induced apoptosis of spheroids in the presence of active NF-κB. We also tested whether both NF-κB activation and active Akt signaling were required for the survival, or whether active Akt alone was sufficient. We blocked NF-B activation in cells expressing constitutively active Akt (Aktmp) by infecting HUVEC with an adenovirus expressing a nondegradable I-κB (AdΔNI-κB - that prevents IkB-NFκB dissociation (Brown et al., Science 267, 1485-1488. (1995)). AdΔNI-*κ*B sensitized fibronectin-adherent HUVEC to TNF±IFNγ-induced apoptosis and this was not affected by Aktmp. Control electroporation (pBS) or adenovirus infection (AdLacZ) had no effect. AdΔNI-κB also sensitized spheroids overexpressing Aktmp to TNF±IFNγ-induced apoptosis (Fig. 4*d*). To test whether Akt could protect against low doses of TNF in HUVEC lacking NF-κB activation, adherent monolayers of wt and Aktmp-expressing HUVEC were infected with AdΔNI-κB and exposed to TNF (0.33 to 100 ng/ml). AdΔNI-κB sensitized HUVEC to apoptosis (TNF > 0.1 ng/ml), but Aktmp did not protect such HUVECS even at these low TNF doses (Fig 4*e*). Furthermore, LY294002 and wortmannin did not inhibit - ICAM-1 expression induced by TNF, indicating that NF-κB activation in HUVEC did not need Akt signaling (Fig. *4f* and data not shown), and consistent with the induction of ICAM-1 in spheroids (see figure 3*c*). By contrast, HUVEC infection with AdΔNI-κB suppressed ICAM-1 expression in response to TNF±IFNγ (Fig. *4f*)
Taken together these results demonstrated that survival of HUVEC exposed to TNF±IFNγ required the simultaneous activation of Akt and NF-κB.

### Example 5: Integrin ligation promotes activation of FKHR and MDM2 and suppresses phosphorylation of MEK, p38 and JNK

The anti-apoptotic activity of Akt was originally ascribed to its phosphorylation and inhibition of caspase-9 and Bad (Datta et al., Genes Dev. 13, 2905-2927. (1999)). Now, however, Akt-dependent survival has been shown to involve phosphorylation and inhibition of Forkhead transcription factors (FKHR/FKHRL1) (Datta et al., Genes Dev. 13, 2905-2927. (1999); Brunet et al., Cell 96, 857-868. (1999)) and of MDM2, p53 degradation (Mayo & Donner, Proc. Natl. Acad. Sci. USA 98, 11598-11603. (2001)), and suppression of activation of the protein kinases ERK, p38 and JNK (Rommel et al., Science 286, 1738-1741. (1999); Gratton et al., J. Biol. Chem. 276, 30359-30365. (2001); Park et al., J. Biol. Chem. 277, 2573-2578. (2002); Madge & Pober, J. Biol. Chem. 275, 15458-15465. (2000)). We investigated whether deficient integrin ligation and Akt signaling were associated with alterations in these signaling pathways. We determined the levels of MDM2, p53, and of phosphorylated FKHR/FRKHL1, MEK, p38 and -JNK in adherent HUVEC and spheroids exposed to TNF/IFNγ. Such spheroids had deficient phosphorylation of FKHR/FKRL1, reduced levels of MDM2 and accumulation of p53, compared to fibronectin-adherent cells (Fig. 5*a*). In addition, spheroids had increased basal and TNF/IFNγ-induced phosphorylation of MEK, p38 and JNK (Fig. 5*b*). These results are consistent with a role of Akt in promoting survival by inhibiting FKHR/FKHRL1, by decreasing p53 levels, and by suppressing phosphorylation of MEK, p38 and JNK.

### Example 6: Inhibition of integrin-dependent adhesion by small molecule compounds sensitizes endothelial cells against TNFα -induced apoptosis in vitro and in vivo.

### Reduction in integrin ligation sensitizes adherent-HUVEC to TNF-induced apoptosis

Increased sensitivity to TNF under conditions of reduced integrin ligation is not unique to spheroids: HUVEC cultured on poly-L-Lysine (PLL), a substrate that promotes integrin-independent adhesion (Bershadsky et al., Curr. Biol. 6, 1279-1289. (1996)) survived on PLL, and addition of TNF±IFNγ caused a massive death (Fig. 6*a*), a death prevented by expression Aktmp (not shown). In addition, we selectively inhibited integrin αVβ3 in HUVEC on fibronectin with EMD121974 ((cyclic(Arg-Gly-Asp-D-Phe-[N-Me]-Val), an αVβ3/αVβ5 antagonistic cyclopeptide) (Dechantsreiter et al., J. Med. Chem. 42, 3033-3040. (1999)) that does not affect the □5□1 component of the α5β1/αVβ3-dependent adhesion on fibronectin (Fig. 6*b*). While neither TNF/IFNγ nor EMD121974 alone affected survival, combined exposure to TNF/IFNγ and EMD121974 (but not a non-inhibitory control peptide EMD135981) increased apoptosis and detachment (Fig. 6*c*), and in reduced survival (Fig. 6*d*). Expression of Aktmp protected fibronectin-adherent HUVEC against apoptosis induced by TNF, IFNγ and EMD121974 (Fig. 6*e*).

### Example 7: Reduction in integrin ligation sensitizes adherent-HUVEC to apoptosis induced by different death ligands of the TNF-ligand family.

Increased sensitivity to pro-apoptotic effects of death receptor signaling upon reduced integrin ligation was not restricted to TNF but was also observed when HUVEC, cultured on fibronectin, were exposed to TRAIL and FasL in the presence of EMD121974. LIGHT, a ligand binding to receptors lacking a death domain, showed no synergism with αVβ3-blockage (Fig. 7).

### Example 8: EMD121974 sensitized established tumors to TNF anti-tumor activity

Angiogenic endothelial cells express αVβ3 integrin and αVβ3-ligation promotes endothelial cell survival (Brooks et al., Cell 79, 1157-1164 (1994); Brooks et al., Science 264, 569-571 (1994)). The observation that EMD121974 sensitized endothelial cells to TNF-induced apoptosis *in vitro,* suggested that this compound could enhance the anti-tumor activity of TNF. To test this hypothesis we treated rats bearing syngeneic the BN175 soft tissue sarcoma, a highly aggressive and vascularized tumor resistant to TNF-cytotoxicity *in vifro* and *in vivo* (Manusama et al., Oncol. Rep. 6, 173-177. (1999)). We used the isolated limb perfusion (ILP) technique to administer TNF, EMD121974, or combination thereof, to tumor-bearing limbs. Treatment with TNF or peptide alone had no impact on tumor growth. Combined administration of TNF and EMD121974, by contrast, caused a complete tumor regression in 50% of the animals and an overall significant reduction of tumor growth (Fig. 8). Local or systemic toxicity was not observed in EMD121974/TNF-treated animals, indicating that EMD121974 selectively sensitized tumors toward TNF cytotoxicity. Since BN175 tumor cells are insensitive to TNF and do not express active αVβ3 integrin as assessed by their poor adhesion to fibrinogen even in the presence of high Mn²⁺, and their low sensitivity to αVβ3 selective inhibitors like EMD 121974 (unpublished observation), we conclude that the potent synergistic anti-tumor effect most probably involves disruption of the tumor vasculature.
Taken together with our in vitro data, this strongly supports the importance of integrin αVβ3 over αVβ3 in this system for controlling endothelial survival.

### Example 9: HUVEC culture and electroporation

HUVEC were prepared and cultured as previously described (Ruegg et al., Nature Med 4, 408-414 (1998)) and used between passages 3 and 7. Complete medium is: M199 (Life technologies, Basel, Switzerland), 10% FCS (Seromed, Berlin, Germany), 12 µg/ml of bovine brain extract (Clonetics-Bio Whittaker, Walkersville, MD, USA), 10 ng/ml human rec. EGF (Peprotech, London, UK), 25 U/ml heparin, 1 µg/ml hydrocortisone (Sigma Chemie), 2 mM L-glutamine, 100 µg/ml streptomycin and 100 U/ml penicillin (Life Technologies). For electroporation, HUVEC were resuspended in complete medium, incubated on ice for 5 minutes with the DNA (20 µg specific plasmid and 5 µg pEGFP-C1) and electroporated with a Gene Pulser (Biorad, Glattbrugg, Switzerland). Electroporated HUVEC were cultured for 48 hours before use. Approx. 80% of the cells expressed EGFP 40 hours after electroporation.

### Example 10 : Spheroid formation

HUVEC were collected by trypsinization, resuspended in complete medium at 1.0x10⁶ cells/ml and 1 ml/well were seeded into 12 wells non-tissue culture plates (Evergreen Scientific, Los Angeles, CA, USA) previously coated with 1% BSA. For aggregation studies, 200 µl of the cell suspension were seeded into 1% BSA-coated microwells of ELISA plates (Maxisorp II, NUNC, Roskilde, Denmark) alone or in the presence of mAbs (10 µg/ml), EDTA (5 mM) or Ca²⁺/EDTA (10/5 mM). Spheroid formation was evaluated at 6 hours and 16 hours. Micrographs were take with a Televal 31 microscope (Carl Zeiss AG, Zürich, Switzerland).

### Example 11: Morphological analysis of spheroid

For morphological evaluation, spheroids were embedded in Epon (Fluka Chemie) and thick sections were stained with 1% Metylene/Azur blue. For immunostaining, frozen spheroid sections were fixed in 4% (Fluka Chemis, Buchs, Switzerland) formaldehyde. After blocking with 1% BSA, sections were sequentially incubated for 1 hour with primary mAb (20 µg/ml) and a Cyan3-labeled GaM antiserum (West Grove, PA, USA). For the TUNEL reaction, frozen spheroids sections were fixed in 4% paraformaledhyde and processed as described (Ruegg et al., I.c.). Spherouids were counterstained with propidium iodide for total DNA content. Sections were viewed on a epifluorescence microscope (Axioskop, Carl Zeiss AG) equipped with a CCD camera (Photonic Science, Milham, UK) or by a laser confocal microscope (LSM 410, Carl Zeiss AG). The apoptosis index was determined by calculating the ratio between the green (TUNEL staining of fragmented DNA) and red (propidium iodide staining by total DNA) pixels. Number of analyzed spheroids per condition were: C, 31; T, 21; TI, 12. For the detection of apoptotic cells in cultures, the DNA dye YoPro-1 (250 nM) was added to the whole culture or to the collected floating cells (Delhase, M., Li, N. & Karin, M. Kinase regulation in inflammatory response. Nature 406, 367-368. (2000)). Cultures were viewed by inverted fluorescence microscopy (Leica DM IRB, Heerbrugg, Switzerland). For electron microscopy, spheroids were fixed with 2.5% glutaraldehyde in 100 mM cacodylate buffer and postfixed in 1 % OsO₄. The cells were dehydrated in ethanol and embedded in Epon. Ultra thin sections were examined using a Philips CM10 transmission electron microscope.

### Example 12: Cell survival and proliferation

For survival, HUVEC spheroids plated at 1x10⁶ cells/ml in 1% BSA-coated 24 mm wells, or adherent cells plated at 4x10⁵ cells in 3 µg/ml fibronectin-coated 35 mm wells of non-tissue culture plates (Evergreen Scientific), were stimulated with TNFα (200 ng/ml=10⁴ U/ml) ± IFNγ (330 ng/ml=10⁴ U/ml). Kinase inhibitors or EMD peptides were added 1 hour or 4 hours before stimulation, respectively at the following concentrations: wortmannin, 100 nM; LY294002, 20 µM; EMD peptides, 300 µM. After 16 hours culture, cells were harvested by dissociation (5 minutes at 20°C for spheroids) with 5 mM EDTA or 1x trypsin (for adherent cultures), washed, resuspended in complete medium at 4x10⁵ cells/ml, aliquoted at 100 µl/well in microtiter tissue culture plates (Falcon, Becton Dickinson) and titrated in 1:2 steps in triplicates. Relative cell number was assessed 48 hours later by measuring MTT conversion during the last 4 hours of culture. Results are given as O.D. values at 540 nm (Packard Spectra Count, Meriden, CT, USA) and represent the mean of triplicate wells ± s.d.

### Example 13: Cell detachment assays

Maxisorp II ELISA plates were coated with 1 µg/well of fibrobnectin or 0.5% gelatin overnight at 4°C in PBS. Coated wells were rinsed and blocked with 1% BSA for 2 hours at 37°C and rinsed before use. 2x10⁴ c/well in basal medium without FCS were added and briefly sedimented by centrifugation (40xg). Cells were let adhere for 2 hours at 37°C before peptides were added at graded concentrations. After 2 hours, wells were rinsed with warm PBS, and attached cells were fixed in 2% paraformaldehyde, stained with 0.5% crystal violet (Sigma Chemie) and quantified by O.D. reading at 620 nm (Packard Spectra Count). Results are given as O.D. values and represent the mean of duplicate wells ± s.d. of specific adhesion ( = adhesion on ECM protein minus adhesion on BSA).

### Example 14: Flow cytometry

Indirect immunostaining of HUVEC and EGFP expression were performed following standard protocol (Ruegg et al., I.c.). Dead cells were excluded by propidium iodide staining. All samples were analyzed with a FACScan II® and Cell Quest® software (Becton Dickinson, Mountain View CA, USA).

### Example 15: Electrophoretic mobility shift assay (EMSA)

Nuclear extracts of HUVEC (1x10⁶ cell per condition) were prepared as described (Cai et al., J Biol Chem 272, 96-101. (1997))and incubated with a synthetic double-stranded 31-mer oligonucleotide containing the kB sequences of the human HIV long terminal repeat end-labeled with [γ-32 P]ATP using the T4 kinase. Binding of NF-κB to the 32 P-labeled oligonucleotide was determined by PAGE and autoradiography.

### Example 16: Western blotting

50 µl of a cell lysate supernatant (1x10⁶ in 250 µl 2x Laemmli Buffer) were resolved by 7.5%-12.5% SDS-PAGE and transferred by wet blotting (Bio Rad) to Immobilon-P membranes (Millipore, Volketswil, Switzerland). Membranes were sequentially incubated in 5% dry milk for 1 hour, with the primary antibody overnight at 4°C, and with a HRP-labeled GaM (Dako, Zug, Switzerland) for 1 hour. The ECL system was used for detection (Amersham-Pharmacia Biotech). For reprobing, membranes were stripped in 2% SDS, 50 mM Tris and100 mM BME, for 30 minutes hour at 50°C.

## Claims

1. A pharmaceutical composition, comprising in a therapeutically effective amount at least
(i) one anti-angiogenic agent, wherein said anti-angiogenic agent is a monoclonal antibody directed against integrin receptor α_{ν}β₃, selected from the group consisting of MAb 14D9.F8, MAb 17.E6, LM609 and Vitaxin, and
(ii) tumor necrosis factor alpha (TNFα),
optionally together with a pharmaceutically acceptable carrier, excipient or diluent.

2. A pharmaceutical composition for use in the treatment tumors and tumor metastases comprising at least
(i) one anti-angiogenic agent, wherein said anti-angiogenic agent is a monoclonal antibody directed against integrin receptor α_{ν}β₃, selected from the group consisting of MAb 14D9.F8, MAb 17.E6, LM609 and Vitaxin, and
(ii) tumor necrosis factor alpha (TNFα),
optionally together with a pharmaceutically acceptable carrier, excipient or diluent.

3. A pharmaceutical composition according to claim 1 or a pharmaceutical composition for use according to claim 2, wherein said anti-angiogenic agent and TNFa are linked together to form one fusion molecule.

4. A pharmaceutical composition according to claim 1, 2 or 3, further comprising at least one cytotoxic and/or chemotherapeutic agent.

5. A pharmaceutical composition according to claim 4, wherein said cytotoxic agent is interferon gamma (IFNγ).

6. A pharmaceutical composition according to claim 4 and/or 5, wherein said chemotherapeutic compound is selected from the group consisting of cisplatin, doxorubicin, gemcitabine, docetaxel, paclitaxel (taxol), bleomycin.

7. A pharmaceutical composition according to one or more of claims 1 to 5, comprising
i) MAb 17.E6, LM609 or Vitaxin,
ii) TNFα, and optionally
iii) IFNγ.

8. A pharmaceutical kit for treating tumors and tumor metastases comprising a package comprising
(i) at least one anti-angiogenic agent, wherein said anti-angiogenic agent is a monoclonal antibody directed against integrin receptor α_{ν}β₃, selected from the group consisting of MAb 14D9.F8, MAb 17.E6, LM609 and Vitaxin;
(ii) TNFα, and optionally
(iii) a cytotoxic and/or chemotherpeutic agent.

9. A pharmaceutical kit according to claim 8, wherein the cytotoxic agent is IFNγ.

10. A pharmaceutical kit according to claim 8 and/or 9 comprising
(i) MAb 17.E6, LM609 or Vitaxin,
(ii) TNFα, and
(iii) IFNγ.

11. A pharmaceutical kit according to one or more of claims 8 to 10, wherein said pharmaceutically active agents are provided in separate containers in said package.

12. Use of
i) at least one monoclonal antibody directed against integrin receptor α_{ν}β₃, selected from the group consisting of MAb 14D9.F8, MAb 17.E6, LM609 and Vitaxin, and
(ii) tumor necrosis factor alpha (TNFα),
for the manufacture of a medicament for simultaneous or sequential administration for treating tumors or tumor metastases in an individual.

13. MAb 17.E6, LM609 or Vitaxin for use in the treatment tumors and tumor metastases in combination with TNFα, optionally together with a pharmaceutically acceptable carrier, excipient or diluent.

14. MAb 17.E6, LM609 or Vitaxin for use according to claim 17, additionally together with IFNγ.

15. MAb 17.E6, LM609 or Vitaxin for use according to claim 17 or 18, additionally together with at least one chemothereapeutic agent selected from the group consisting of cisplatin, doxorubicin, gemcitabine, docetaxel, paclitaxel (taxol), bleomycin and melphalan.
